# EUROPEAN PATENT APPLICATION

(11) **EP 1 921 072 A1**
(43) Date of publication of application: **14.05.2008**
(21) Application number: 06380290.4
(22) Date of filing: 10.11.2006
(51) Int. Cl.: C07D 249/06, A61P 3/00, A61K 31/4192

(54) **1,2,3-Triazole derivatives as cannabinoid-receptor modulators**

(71) Applicant: Laboratorios del Dr. Esteve S.A., 08041 Barcelona (ES)
(72) Inventor: Jagerovic, Nadina, I. Quimico Médica, 28006 Madrid (IT); Gomez-de la Olivia, Cristina Ana, I Quimico Médica, 28006 Madrid (IT); Goya-Laza, Maria Pilar, I. Quimico Médica, 28006 Madrid (IT); Dordal Zueras, Alberto, 08041 Barcelona (ES); Cuberes-Altisent, Maria, Rosa, 08041 Barcelona (ES)
(74) Representative: ABG Patentes, S.L.

(57) **Abstract**

The invention relates to the use of compounds having pharmacological activity towards the so-called cannabonid receptors, and more particularly to some 1,2,3-triazole derivatives of formula (I): 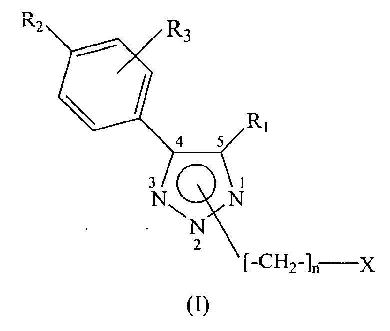 to processes of preparation of such compounds and to pharmaceutical compositions comprising them.

## Description

### FIELD OF THE INVENTION

The present invention relates to the use of compounds having pharmacological activity towards the so-called cannabonid receptors, and more particularly to some 1,2,3-triazole derivatives, to processes of preparation of such compounds and to pharmaceutical compositions comprising them.

### BACKGROUND OF THE INVENTION

Cannabinoids are compounds, which are derived from the cannabis sativa plant which is commonly known as marijuana. The most active chemical compound of the naturally occurring cannabinoids is tetrahydrocannabinol (THC), particularly Δ⁹-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated CB₁ and CB₂ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the CB₁-Receptor is involved in many different food-intake related disorders such as bulimia or obesity, including obesity associated with type 11 diabetes (non-insulin-dependent diabetes) and thus, compounds suitable for regulating this receptor may be used in the prophylaxis and/or treatment of these disorders.

In this sense, many compounds which can be used as cannabinoid receptor modulators have been developed in the recent years. For example US2006/0205720 relates to bicyclic pyrazolyl and imidazolyl compounds as cannabnoid receptor ligands, in particular as CB₁ receptor antagonists. EP1696930 refers to tricyclic 1-[(3-indol-3-yl) carbonyl] piperazine derivatives as CB₁ receptor agonists directed preferably to the treatment of pain and spasticity associated with multiple sclerosis. WO2005/061509 relates to azabicyclic heterocycles as CB₁ modulators and WO2006/084975 describes the antagonist properties of some N-[(4,5-diphenyl-2-thienyl) methyl] amine derivatives towards CB₁ receptor.

With regard to the chemical structure of the compounds described in the present patent application, it is to be highlighted that the 1,2,3-triazole ring system has been the subject of considerable research mainly due to the pharmacological properties shown by some of its derivatives and also because of its usefulness in synthetic organic chemistry. Among the first, recent reports have dealt with 1,2,3-triazoles as antimicrobial agents, as potassium channel activators (Calderone, V. et al. Eur. J. Med. Chem., 2005, 40, 521-528). 1,2,3-Triazole derivatives of glycosyl and galactoside have been respectively described as glycosidase (Rossi, L.L. et al., Bioorg. Med. Chem. Lett., 2005, 15, 3596-3599) and galactin-3 (Salameh, A. et al., Bioorg. Med. Chem. Lett., 2005, 15, 3344-3346) inhibitors. 2-Pyridinyl-1,2,3-triazoles have been described as transforming growth factor beta 1 type 1 receptor (Kim, J. et al, Bioorg. Med. Chem. Lett, 2004, 14, 2401-2405). In addition, a 1,2,3-triazole-4-carboxamide derivative (CAI) has been identified as an orally bioavailable calcium influx and signal transduction inhibitor with anti-angiogenic and anti-metastatic properties in different human tumours (Perabo, F.G., et al., Anticancer Res., 2005, 25, 725-729). The 1,2,3-triazole moiety has been identified as an effective replacement for a peptide group in HIV-1 protease inhibitors (Brik, J. et al., Chembiochem, 2005, 6, 1167-1169). Concerning synthetic issues, 1,2,3-triazoles can be considered the ideal representatives of "click chemistry" (Kolb, C. et al., Angew. Chem. Int. Edit., 2001, 40, 2004-2021), and recently, 1,2,3-triazole has been used as a safer and practical alternative to cyanide in Bruylants reaction (Prashad M. et al., Tetrahedron Lett., 2005, 46, 5455-5458).

However, none of these documents suggests the effect of these compounds on the cannabinoid receptors.

There is still a need to find compounds that have pharmacological activity towards cannabinoid receptors, more particularly compounds suitable for the modulation of cannabinoid 1 receptors, being both effective and selective, and having good "drugability" properties, i.e. good pharmaceutical properties related to administration, distribution, metabolism and excretion.

### SUMMARY OF THE INVENTION

We have now found a family of structurally distinct triazole derivatives which are particularly suitable for the modulation of cannabinoids receptors, more particularly for the modulation of cannabinoid 1 (CB1) receptors. These compounds have a high affinity for these receptors and they can act as modulators, e.g. antagonists, inverse antagonists or agonists on said receptors. They are therefore suitable for the treatment and/or profilaxis of various disorders related to the central nervous system, the immune system, the cardiovascular system, the endocrinous system, the respiratory system, the gastrointestinal tract or reproduction in humans and/or animals.

In one aspect the invention is directed to the use of a compound of the formula I: 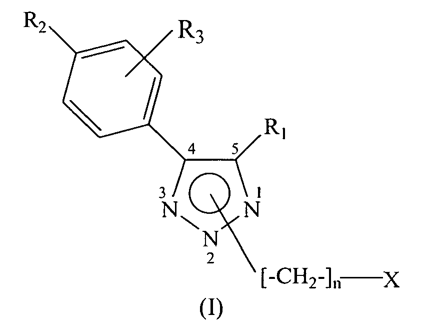 wherein:
R₁ is selected from hydrogen and substituted or unsubstituted aryl;
R₂ is selected from halogen, lower alkyl and lower alkoxy;
R₃ is selected from hydrogen and halogen;
X is selected from hydrogen, substituted or unsubstituted cycloalkyl, substituted or unsubstituted aryl, COOR' and CONHR"; wherein R' is a lower alkyl and R" is a cycloalkyl or heterocyclyl group;
n is selected from 0, 1, 2, 3, 4, 5, 6, 7, and 8,
or a pharmaceutically acceptable salt, isomer, prodrug or solvate thereof, in the manufacture of a medicament for the treatment or prophylaxis of cannabinoid receptor mediated disease or condition.

In a preferred embodiment the compound of formula I is used in the manufacture of a medicament for the treatment or profilaxis of the central nervous system, disorders of the immune system, disorders of the cardiovascular system, disorders of the endocrinous system, disorders of the respiratory system, disorders of the gastrointestinal tract or reproductive disorders. Preferably, the medicament is for the treatment or profilaxis of psychosis.

In another preferred embodiment the compound of formula I is used in the manufacture of a medicament for the treatment or profilaxis of food intake disorders, preferably bulimia, anorexia, cachexia, obesity, type II diabetus mellitus (non-insuline dependent diabetes mellitus), more preferably obesity.

In another preferred embodiment the compound of formula I is used in the manufacture of a medicament for the treatment or profilaxis of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Still in another preferred embodiment, the compound of formula I is used in the manufacture of a medicament for the treatment or profilaxis of cancer, preferably selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the group consisting of colon cancer, bowel cancer and prostate cancer.

In another preferred embodiment, the compound of formula I is used in the manufacture of a medicament for the treatment or profilaxis of a disorder selected from the group consisting of bone disorders, preferably osteoporosis, more preferably osteoporosis associated with a genetic predisposition, sex hormone deficiency or ageing, cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus and pain.

In another preferred embodiment the compound of formula I is used in the manufacture of a medicament tor potentiating the analgesic effect of narcotic and non-narcotic analgesics and for influencing intestinal transit.

In a second aspect the invention is directed to a compound of formula (I): 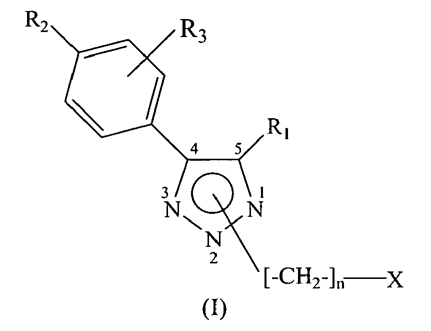 wherein:
R₁ is selected from hydrogen and substituted or unsubstituted aryl;
R₂ is selected from halogen, lower alkyl and lower alkoxy;
R₃ is selected from hydrogen and halogen;
X is selected from hydrogen, substituted or unsubstituted cycloalkyl, substituted or unsubstituted aryl, COOR' and CONHR"; wherein R' is a lower alkyl and R" is a cycloalkyl or heterocyclyl group;
n is selected from 0, 1, 2, 3, 4, 5, 6, 7, and 8,
or a pharmaceutically acceptable salt, isomer, prodrug or solvate thereof, with the proviso that formula (I) is not:
4-(4-isopropoxy-phenyl)-2-ethyl-2*H*-[1,2,3]triazole;
4-(4-hexyloxy-phenyl)-2-ethyl-2*H*-[1,2,3]triazole;
4-(4-chloro-phenyl)-2-ethyl-2*H*-[1,2,3]triazole;
4-(4-methoxy-phenyl)-2-methyl-2*H*-[1,2,3]triazole;
4-(4-chloro-phenyl)-2-methyl-2*H*-[1,2,3]triazole;
4-(4-bromo-phenyl)-2-methyl-2*H*-[1,2,3]triazole;
4-(4-fluoro-phenyl)-2-methyl-2*H*-[1,2,3]triazole
4-(3,4-dichloro-phenyl)-2-methyl-2*H*-[1,2,3]triazole;
4-(2,4-difluoro-phenyl)-2-methyl-2*H*-[1,2,3]triazole;
4-(4-bromo-phenyl)-1-ethyl-1*H*-[1,2,3]triazole;
4-(4-isopropoxy-phenyl)-1-ethyl-1*H*-[1,2,3]triazole;
4-(4-butoxy-phenyl)-1-ethyl-1*H*-[1,2,3]triazole;
4-(4-hexyloxy-phenyl)-1-ethyl-1*H*-[1,2,3]triazole;
4-(4-bromo-phenyl)-1-methyl-1*H*-[1 ,2,3]triazole.

In one embodiment R₁ is preferably hydrogen, phenyl or 4-methyl-phenyl.

In another embodiment R₂ is preferably chloro, bromo, fluor, methyl or methoxy.

In another embodiment R₃ is preferably hydrogen, chloro or fluor.

Further in another preferred embodiment X is hydrogen, adamanthyl, substituted or unsubstituted phenyl, COOR' or CONHR" wherein R' is ethyl and R" is preferably cyclohexyl, pyridine or morpholine.

In a third aspect the invention is directed to a process for the preparation of a compound of formula I or a salt, isomer, prodrug or solvate thereof.

Finally, in another aspect the invention is directed to a pharmaceutical composition which comprises a compound as defined above or a pharmaceutically acceptable salt, isomer, prodrug or solvate thereof, and a pharmaceutically acceptable carrier, adjuvant or vehicle.

The above mentioned preferences and embodiments can be combined to give further preferred compounds or uses.

### DETAILED DESCRIPTION OF THE INVENTION

The typical compounds of this invention effectively and selectively modulate the cannabinoid-1 receptor.

In the present description the following terms have the meaning indicated:
"Lower alkyl" refers to a straight or branched hydrocarbon chain radical consisting of carbon and hydrogen atoms, containing no unsaturation, having one to six carbon atoms, and which is attached to the rest of the molecule by a single bond, e. g., methyl, ethyl, propyl, isopropyl, n-butyl, sec-butyl, tert-butyl, pentyl, hexyl, etc.
"Lower alkoxy" refers to a radical of the formula -ORa where Ra is a lower alkyl radical as defined above, e. g., methoxy, ethoxy, propoxy, etc.
"Halo" refers to bromo, chloro, iodo or fluoro.
"Cycloalkyl" refers to a stable 3-to 10-membered monocyclic or bicyclic radical which is fully saturated, and which consists solely of carbon and hydrogen atoms, such as cyclohexyl or adamantyl. Unless otherwise stated specifically in the specification, the term "cycloalkyl" is meant to include cycloalkyl radicals which are optionally substituted by one or more substituents such as alkyl, halo, hydroxy, amino, cyano, nitro, alkoxy, carboxy, alkoxycarbonyl, etc.
"Aryl" refers to single and multiple ring radicals, including multiple ring radicals that contain separate and/or fused aryl groups. Typical aryl groups contain from 1 to 3 separated or fused rings and from 6 to about 18 carbon ring atoms, such as phenyl, naphthyl, indenyl, fenanthryl or anthracyl radical. The aryl radical may be optionally substituted by one or more substituents such as hydroxy, mercapto, halo, alkyl, phenyl, alkoxy, haloalkyl, nitro, cyano, dialkylamino, aminoalkyl, acyl, alkoxycarbonyl, etc.
"Heterocyclyl" refers to a substituted or unsubstituted stable 3-to 8 membered ring radical which consists of carbon atoms and from one to three heteroatoms selected from the group consisting of nitrogen, oxygen and sulfur, preferably a 4-to 7-membered ring with one or more heteroatoms, more preferably a 5, 6 or 7-membered ring with one or more heteroatoms. It may be partially of fully saturated or aromatic. Additionally, the heterocycle may be also monocyclic, bicyclic or tricyclic ring system, which may include fused ring systems; and the nitrogen, carbon or sulfur atoms in the heterocyclyl radical may be optionally oxidised; the nitrogen atom may be optionally quatemized. Examples of such heterocycles include, but are not limited to, azepines, benzimidazole, benzothiazole, furan, isothiazole, imidazole, indole, piperidine, piperazine, purine, quinoline, thiadiazole, tetrahydrofuran, coumarine, morpholine, pyrrole, pyrazole, oxazole, isoxazole, triazole, imidazole, etc.

The heterocyclyl group may be substituted at one or more available positions by one or more suitable groups, e. g., halogen such as fluoro, chloro, bromo and iodo; cyano; hydroxyl; nitro; alkyl groups including those groups having 1 to about 12 carbon atoms or from 1 to about 6 carbon atoms and more preferably 1-3 carbon atoms; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

The 1,2,3-triazole derivatives used in the invention are distinguished by a broad spectrum of beneficial effects, while at the same time showing relatively little undesired effects, i.e. effects which do not positively contribute to or even interfere with the well being of the patient.

Thus, one aspect of this invention relates to a method of treating or preventing a cannabinoid receptor mediated disease or condition which method comprises administering to a patient in need of such a treatment a therapeutically effective amount of a compound of formula I or a pharmaceutical composition thereof. Among the cannabinoid receptor mediated diseases that can be treated or prevented are diseases of the central nervous system, disorders of the immune system, disorders of the cardiovascular system, disorders of the endocrinous system, disorders of the respiratory system, disorders of the gastrointestinal tract or reproductive disorders. Preferably, the method is particularly suitable for the treatment or prophylaxis of psychosis.

In an embodiment of the invention, the cannabinoid receptor mediated diseases that can be treated or prevented are food intake disorders, preferably bulimia, anorexia, cachexia, obesity, type II diabetus mellitus, more preferably obesity.

In still another embodiment cannabinoid receptor mediated disease that can be treated or prevented is alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

In another embodiment, cannabinoid receptor mediated disease that can be treated or prevented is cancer, preferably selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the group consisting of colon cancer, bowel cancer and prostate cancer.

Further, in another embodiment cannabinoid receptor mediated disease that can be treated or prevented is a disorder selected from the group consisting of bone disorders, preferably osteoporosis, more preferably osteoporosis associated with a genetic predisposition, sex hormone deficiency or ageing, cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus and pain.

Another embodiment of this invention relates to a method of treating for potentiating the analgesic effect of narcotic and non-narcotic analgesics and for influencing intestinal transit.

Preferred compounds of formula I used in the invention are the following:
4-(*p*-tolyl)-1*H*(2*H*)-[1,2,3]triazole
4-(*p*-methoxyphenyl)-1*H*(2*H*)-[1,2,3]triazole
4-(4-bromo-2-fluorophenyl)-1*H*(2*H*)-[1,2,3]triazole
4-(*p*-chlorophenyl)-5-phenyl-1*H*(2*H*)-[1,2,3]triazole
4-(2,4-dichlorophenyl)-5-*p*-tolyl-1*H*(2*H*)-[1,2,3]triazole
2-Pentyl-4-*p*-tolyl-2*H*-[1,2,3]triazole
1-Pentyl-4-*p*-tolyl-1*H*-[1,2,3]triazole
1-Pentyl-5-*p*-tolyl-1*H*-[1,2,3]triazole
4-(*p*-Methoxyphenyl)-2-pentyl-2*H*-[1,2,3]triazole
4-(*p*-Methoxyphenyl)-1-pentyl-1*H*-[1,2,3]triazole
4-(4-Bromo-2-fluorophenyl)-2-pentyl-2*H*[1,2,3]triazole
4-(4-Bromo-2-fluorophenyl)-1-pentyl-1*H*-[1,2,3]triazole
4-(*p*-Chlorophenyl)-2-pentyl-5-phenyl-2*H*-[1,2,3]triazole
4-(*p*-Chlorophenyl)-1-pentyl-5-phenyl-1*H*-[1,2,3]triazole
5-(*p*-Chlorophenyl)-1-pentyl-4-phenyl-1*H*-[1,2,3]triazole
4-(2,4-Dichlorophenyl)-2-pentyl-5-*p*-tolyl-2*H*-[1,2,3]triazole
4-(*p*-Chlorophenyl)-2-heptyl-5-phenyl-2*H*-[1,2,3]triazole
4-(*p*-Chlorophenyl)-1-heptyl-5-phenyl-1*H*-[1,2,3]triazole
5-(*p*-Chlorophenyl)-1-heptyl-4-phenyl-1*H*-[1,2,3]triazole
2-(*p*-Bromobenzyl)-4-(*p*-chlorophenyl)-5-phenyl-2*H*-[1,2,3]triazole
1-(*p*-Bromobenzyl)-4-(*p*-chlorophenyl)-5-phenyl-1*H*-[1,2,3]triazole
1-(*p*-Bromobenzyl)-5-(*p*-chlorophenyl)-4-phenyl-1*H*-[1,2,3]triazole
2-Benzyl-4-(*p*-chlorophenyl)-5-phenyl-2*H*-[1,2,3]triazole
1-Benzyl-4-(*p*-chlorophenyl)-5-phenyl-1*H*-[1,2,3]triazole
1-Benzyl-5-(*p*-chlorophenyl)-4-phenyl-1*H*-[1,2,3]triazole
2-Benzyl-4-(4-bromo-2-fluorophenyl)-2*H*-[1,2,3]triazole
1-Benzyl-4-(4-bromo-2-fluorophenyl)-1*H*-[1,2,3]triazole
4-(4-Bromo-2-fluorophenyl)-2-(ethoxycarbonyl)methyl-2*H*[1,2,3]-triazole
4-(4-Bromo-2-fluorophenyl)-1-(ethoxycarbonyl)methyl-1*H*-[1,2,3]triazole
4-(*p*-Chlorophenyl)-2-(ethoxycarbonyl)methyl-5-phenyl-2*H*-[1,2,3]triazole
2-Adamantyl-4-(4-bromo-2-fluorophenyl)-2*H*-[1,2,3]triazole
1-Adamantyl-4-(4-bromo-2-fluorophenyl)-1*H*-[1,2,3]triazole
2-Adamantyl-4-(*p*-chlorophenyl)-5-phenyl-2*H*-[1,2,3]triazole
2-Adamantyl-4-(2,4-dichlorophenyl)-5-*p*-tolyl-2*H*-[1,2,3]triazole
4-(4-Chlorophenyl)-5-phenyl-2-(piperidin-1-yl-carbamoyl)methyl-2*H*-[1,2,3]triazole
4-(4-Bromo-2-fluorophenyl)-2-(piperidin-1-yl-carbamoyl)methyl-2*H*-[1,2,3]triazole
4-(4-Chlorophenyl)-5-phenyl-2-(morpholin-4-yl-carbamoyl)methyl-2*H*-[1,2,3]triazole
4-(4-Bromo-2-fluorophenyl)-2-(morpholin-4-yl-carbamoyl)methyl-2*H*-[1,2,3]triazole
4-(4-Chlorophenyl)-5-phenyl-2-(cyclohexyl-carbamoyl)methyl-2*H*-[1,2,3]triazole
4-(4-Bromo-2-fluorophenyl)-2-(cyclohexyl-carbamoyl)methyl-2*H*-[1,2,3]triazole
or a pharmaceutically acceptable salt, isomer, prodrug or solvate thereof.

Unless otherwise stated, the compounds used in the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The term "pharmaceutically acceptable salts, isomers, solvates, prodrugs" refers to any pharmaceutically acceptable salt, isomer, solvate, prodrug or any other compound which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts, isomers, solvates, prodrugs and derivatives can be carried out by methods known in the art.

For instance, pharmaceutically acceptable salts of compounds provided herein are synthesized from the parent compound which contains a basic moiety by conventional chemical methods. Generally, such salts are, for example, prepared by reacting the free base forms of these compounds with a stoichiometric amount of the appropriate acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate.

Particularly favored derivatives or prodrugs are those that increase the bioavailability of the compounds used in this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

Any compound that is a prodrug of a compound of formula I is within the scope of the invention. The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides.

The compounds used in the invention may be in crystalline form either as free compounds or as solvates and it is intended that both forms are within the scope of the present invention. Methods of solvation are generally known within the art. Suitable solvates are pharmaceutically acceptable solvates. In a particular embodiment the solvate is a hydrate.

The compounds of formula I, their salts, isomers, prodrugs or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula I, or of its salts, isomers, solvates or prodrugs.

The compounds used in the present invention represented by the above described formula (I) may include enantiomers depending on the presence of quiral centres. The single isomers, enantiomers or diastereoisomers and mixtures thereof fall within the scope of the present invention.

The compounds of formula I defined above can be obtained by available synthetic procedures. In an embodiment of the invention, the process to prepare the compound of formula (I) comprises the following step:
a) cycloaddition of tri-n-butyltin azide with a compound of formula (II): 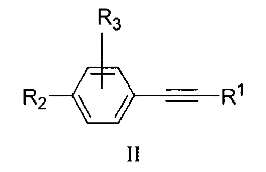 to give a compound of formula (III): 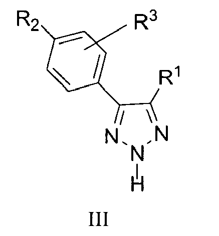
wherein
R₁ is selected from hydrogen and substituted or unsubstituted aryl;
R₂ is selected from halogen, lower alkyl and lower alkoxy;
R₃ is selected from hydrogen and halogen;

This compound (III) corresponds to the compound of formula (I) when n=0 and X=H.

In this reaction, the *NH*-1,2,3-triazoles (III) bearing the desired substituents R₁, R₂ and R₃, were achieved by cycloaddition of tri-n-butyltin azide with mono- or disubstituted alkynes (II) under pressure and heating conditions according to known methods (S. Kozima, T. Itano, N. Mihara, K. Sisido, T. Isida, J. Organomet. Chem. 1972, 44, 117-126; T. Hitomi, S. Kozima, J. Organomet. Chem. 1977, 127, 273-280). Then, the tributylstannyl group is subsequently replaced by a proton under mild conditions.

The mono- or disubstituted alkynes (II) used in this synthesis may be obtained from commercial sources or may be prepared from the corresponding iodobenzene and monosubstituted alkyne (J. F. Nguefack, V. Bolitt, D. Sinou, Tetrahedron Lett. 1996, 37, 5527-5530) according to the reaction as shown below:

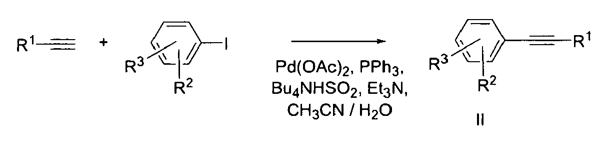

Additionally, the process for the preparation of a compound of formula (I) further comprises the alkylation of the compound of formula (III), previously obtained, with a compound of formula (IV): 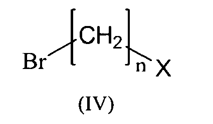 wherein
X is selected from hydrogen, substituted or unsubstituted cycloalky, substituted or unsubstituted aryl, COOR' and CONHR", wherein R' is a lower alkyl and R" is a cycloalkyl or heterocyclyl group; and
n is selected from 0, 1, 2, 3, 4, 5, 6, 7, and 8,
with the proviso that when n=0, X is not hydrogen,
to give a compound of formula (I).

This reaction is usually carried out under basic conditions, for example in the presence of hydroxides such as potassium hydroxide, using a phase transfer catalyst, such as for example BuNBr, as shown in the following scheme:

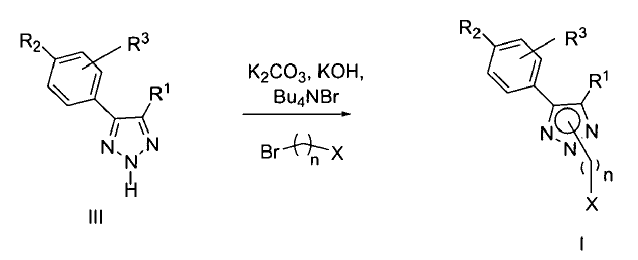

Additionally, the compound of formula (I) wherein X=CONHR" may also be obtained from the compound of formula (I) wherein X=COOR' previously obtained, by reaction with the amine NH₂R", wherein R' is a lower alkyl and R" is a cycloalkyl or heterocyclyl group. Preferably, this reaction is carried out in the presence of trimethylaluminium, such as shown in the following scheme:

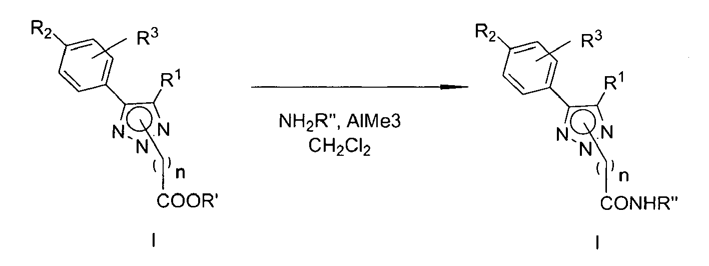

The obtained products obtained in the different reactions may, if desired, be purified by conventional methods, such as crystallisation, chromatography and trituration. Where the above described processes for the preparation of compounds of the invention give rise to mixtures of stereoisomers, these isomers may be separated by conventional techniques such as preparative chromatography. If there are chiral centers the compounds may be prepared in racemic form, or individual enantiomers may be prepared either by enantiospecific synthesis or by resolution.

One preferred pharmaceutically acceptable form is the crystalline form, including such form in pharmaceutical composition. In the case of salts and solvates the additional ionic and solvent moieties must also be non-toxic. The compounds of the invention may present different polymorphic forms, it is intended that the invention encompasses all such forms.

The present invention further provides pharmaceutical compositions comprising a compound of formula (I) of this invention, or a pharmaceutically acceptable salt, isomer, prodrug or solvate thereof together with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the apropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

The following examples are given only as further illustration of the invention, they should not be taken as a definition of the limits of the invention.

### EXAMPLES

### SYNTHESIS

### Materials and methods

All starting materials were commercially available research grade chemicals and used without further purification. Dichloromethane was distilled over CaCl₂. Silical gel 60 F₂₅₄ (Merck) was used for TLC, and the spots were detected with UV light (254 nm). Flash column chromatography was carried out on silica gel 60 (Merck). Melting points were determined on a *Reichert Jung Thermovar* apparatus and are uncorrected. ¹H NMR spectra were recorded on a *Varian Gemini* [200 MHz (¹H), 50 MHz (¹³C)] spectrometer, *Varian Inova 300* or *400* [(300 MHz (¹H), 75 MHz (¹³C) or 400 MHz (¹H), 100 MHz (¹³C)] spectrometer and *Varian Unity 500* [500 MHz (¹H), 125 MHz (¹³C)] spectrometer with TMS as internal reference. Multiplicity were denoted by s (singlet), sw (wide single), d (doublet), t (triplet), q (quartet), quin (quintet), dd (double doublet) and m (multiplet). Mass spectra were determined on a *MSD-Serie 1100 Hewlett Packard* apparatus. Microanalyses were performed with a *Heraeus CHN-O Rapid* analysis in our Analytical Services of Centro de Quimica OrgAnica "Manuel Lora Tamayo" (CSIC).

### Synthesis of 2,4-chloro-1-p-tolylethynylbenzene (compound 1e)

To a mixture of palladium diacetate (42.6 mg, 0.19 mmol), triphenylphosphine (99.7 mg, 0.38 mmol), CH₃CN (8.6 mL) and H₂O (0.9 mL) in a Schlenk tube was added under nitrogen a well-stirred mixture of 1-ethynyl-4-methylbenzene (0.24 mL, 1.9 mmol), 1,4-dichloro-2-iodobenzene (0.51 mL, 3.8 mmol), triethylamine (0.66 mL, 4.7 mmol), tetrabutylammonium hydrogen sulfate (645.1 mg, 1.9 mmol), CH₃CN (8.6 mL) and H₂O (0.9 mL). The reaction was stirred at room temperature for 3 h. The mixture was then hydrolyzed by H₂O (15 mL) and extracted with Et₂O (3 X 15 mL). The combined organic extracts were dried over MgSO₄ Evaporation of the solvent under reduced pressure afforded a solid, which was purified by column chromatography (cyclohexane) and recrystallized from cyclohexane to give the alkyne **1e.** White solid (491.2 mg, 99%); m.p. 69-71 °C. ¹H NMR (200 MHz, CDCl₃, 25 °C): δ = 7.57 (d, ⁴*J*_{3,5} = 2.6 Hz, 1 H, H-3), 7.51 (d, ³*J*_{o,m} = 8.2 Hz, 2 H, H*ₒ*), 7.37 (d, ³*J*_{5,6} = 8.5 Hz, 1 H, H-6), 7.24 (dd, ³*J*_{5,6} = 8.5 Hz, ⁴*J*_{3,5} = 2.6 Hz, 1 H, H-5), 7.21 (d, ³*J*_{o,m} = 8.2 Hz, 2 H, H*ₘ*), 2.42 (s, 3 H, CH₃) ppm; ¹³C NMR (50 MHz, CDCl₃, 25 °C): δ = 139.1 (C-2), 134.0 (C*ₚ*), 132.4 (C-6), 132.1 (C-4), 131.6 (C*ₒ*), 130.1 (C-3), 129.0 (C*ₘ*), 128.9 (C-5), 124.7 (C-1), 119.2 (C*ᵢₚₛₒ*), 95.9 (C-1'), 84.3 (C-2'), 21.4 (CH₃) ppm. EI-MS: m/z (%) = 264 (21), 262 (88), 260 (100) [M⁺]; 225 (30) [M⁺-35]; 189 (75) [M⁺-71]. C₁₅H₁₀Cl₂ (261.15): caldc. C 68.99, H 3.86; found C 69.07, H 4.12.

### Synthesis of triazoles 2-6. General procedure

A mixture of tri-*n*-butyltin azide (0.86 mL, 3.15 mmol) with the appropriate alkyne **1** (3 mmol) was heated at 150 °C for 70 h in a sealed glass bottle. The resulting solution was purified by column chromatography (cyclohexane/AcOEt, 5:1) and recrystallized from (cyclohexane/AcOEt) to give the desired triazoles.

**4-(*p*-tolyl)-1*H*(2*H*)-[1,2,3]triazole (2):** Yellow solid (191.0 mg, 40%); m.p. 139-141 °C. ¹H NMR (200 MHz, DMSO-*d₆*, 25 °C): δ = 14.91 (sw, 1 H, NH), 8.08 (s, 1 H, H-5), 7.61 (d, ³*J*_{o,m} = 7.8 Hz, 2 H, H*ₒ*), 7.11 (d, ³*J*_{o,m} = 7.8 Hz, 2 H, H*ₘ*), 2.21 (s, 3 H, CH₃) ppm; ¹³C NMR (75 MHz, DMSO-*d₆,* 25 °C): δ = 147.4 (C-4), 137.7 (C*ₚ*), 129.3 (C*ₘ*, C-5), 129.1 (C*ᵢₚₛₒ*), 125.4 (C*ₒ*), 20.8 (CH₃) ppm. ES-MS: m/z (%) = 182 [M⁺+Na]; 160 [M⁺+1]. C₉H₉N₃ (159.08): caldc. C 67.90, H 5.70, N 26.40; found C 67.81, H 5.75, N 26.49

**4-(*p*-methoxyphenyl)-1*H*(2*H*)-[1,2,3]triazole (3):** Yellow solid (157.7 mg, 30%); m.p. 163-165 °C. ¹H NMR (200 MHz, CDCl₃, 25 °C): δ = 7.91 (s, 1 H, H-5), 7.76 (d, *³J*_{o,m} = 8.0 Hz, 2 H, H*ₒ*), 7.00 (d, ³*J*_{o,m} = 8.0 Hz, 2 H, H*ₘ*), 3.87 (3 H, s, CH₃) ppm; ¹³C NMR (75 MHz, CDCl₃, 25 °C): δ = 159.5 (C*ₚ*), 147.5 (C-4), 126.5 (C-5), 127.3 (C*ₒ*), 123.1 (C*ᵢₚₛₒ*), 114.7 (C*ₘ*), 55.5 (CH₃) ppm. ES-MS: m/z (%) = 198 [M⁺+Na]; 177 [M⁺+2]; 176 [M⁺+1]. C₉H₉N₃O (175.07): caldc. C 61.70, H 5.18, N 23.99; found C 61.85, H 5.15, N 24.09

**4-(4-bromo-2-fluorophenyl)-1*H*(2*H*)-[1,2,3]triazole (4):** Yellow solid (477.1 mg, 66%); m.p. 139-141 °C. ¹H NMR (200 MHz, DMSO-*d*₆, 25 °C): δ = 11.33 (sw, 1 H, NH), 8.13 (s, 1 H, H-5), 7.87 (dd, ³*J*_{5',6'}= 8.3 Hz, ⁴*J*_{6',F} = 8.1 Hz, 1 H, H-6'), 7.57 (dd, ³*J*_{3',F} = 10.6 Hz, ⁴*J*_{3',5'} = 2.0 Hz, 1 H, H-3'), 7.41 (ddd, ³*J*_{5',6'} = 8.3 Hz, ⁴*J*_{3',5'} = 2.0 Hz, ⁵*J*_{5',F} = 0.6 Hz, 1 H, H-5') ppm; ¹³C NMR (75 MHz, DMSO-*d*₆, 25 °C): δ = 158.9 (d, ¹*J*_{2',F} = 252.5 Hz, C-2'), 132.3 (C-4), 129.6 (C-5), 128.6 (C-6'), 128.5 (C-5'), 121.5 (C-4'), 119.8 (d, ²*J*_{3',F} = 38.8 Hz, C-3'), 118.1 (C-1') ppm. ES-MS: m/z (%) = 244, 242 [M⁺+1]. C₈H₅BrFN₃ (242.05): caldc. C 39.70, H 2.08, N 17.36; found C 39.82, H 2.12, N 17.15

**4-(*p*-chlorophenyl)-5-phenyl-1*H*(2*H*)-[1,2,3]triazole (5):** White solid (413.2 mg, 54%); m.p. 124-126 °C. ¹H NMR (200 MHz, CDCl₃, 25 °C): δ = 7.51-7.46 (m, 4 H, H*ₒ*, H_{*o*'}), 7.38-7.35 (m, 3 H, H_{*m*'}, H_{*p*'}), 7.31 (dt, ³*J*_{o,m} = 8.6 Hz, ⁴*J*_{m,m} = ⁵*J*_{o,m} = 2.2 Hz, 2 H, H*ₘ*) ppm; ¹³C NMR (50.5 MHz, CDCl₃, 25 °C): δ = 142.4 (C-5), 142.0 (C-4), 134.6 (C*ₚ*), 129.6 (C_{*ipso*'}), 129.4 (C*ₒ*), 128.9 (C*ₘ*, C_{*p*'}), 128.8 (C_{*m*'}), 128.6 (C*ᵢₚₛₒ*), 128.2 (C_{*o*'}) ppm. ES-MS: m/z (%) = 278 [M⁺+Na], 256 [M⁺]. C₁₄H₁₀ClN₃ (255.70): caldc. C 65.76, H 3.94, N 16.43; found C 65.93, H 3.84, N 16.37.

**4-(2,4-dichlorophenyl)-5-*p*-tolyl-1*H*(2*H*)-[1,2,3]triazole (6):** White solid (611.1 mg, 67%); m.p. 54-56 °C. ¹H NMR (200 MHz, CDCl₃, 25 °C): δ = 13.56 (sw, 1 H, NH), 7.45 (d, ⁴*J*_{3',5'} = 2.2 Hz, 1 H, H-3'), 7.39 (d, ³*J*_{5',6'} = 8.3 Hz, 1 H, H-6'), 7.36-7.33 (m, 3 H, H-5', H*ₒ*), 7.12 (d, ³*J*_{o,m} = 7.8 Hz, 2 H, H*ₘ*), 2.26 (s, 3 H, CH₃) ppm; ¹³C NMR (75 MHz, CDCl₃, 25 °C): δ = 144.2 (C-5), 140.4 (C-4), 138.7 (C*ₚ*), 132.7 (C-2'), 132.6 (C-4'), 131.8 (C-3'), 131.7 (C-1'), 131.1 (C-6'), 130.3 (C-5'), 129.5 (C*ₘ*), 126.8 (C*ₒ*), 126.3 (C*ᵢₚₛₒ*), 21.4 (CH₃) ppm. EI-MS: m/z (%) = 307 (14), 305 (70), 303 (89) [M⁺]; 270 (46), 268 (100) [M⁺-35]; 233 (47) [M⁺-70]; 213 (60) [M⁺-90]. C₁₅H₁₁Cl₂N₃ (304.03): caldc. C 59.23, H 3.65, N 13.81; found C 59.65, H 3.85, N 13.09.

### Synthesis of N-alkyl-1,2,3-triazoles, N-benzyl-1,2,3-triazoles and N-alkylacetate-1,2,3-triazoles (compounds 7-17) by N-alkylation of compounds 2-6. General procedure.

To a solution of 1,2,3-triazole **2-6** (0.5 mmol) in acetonitrile (3 mL) was added K₂CO₃ (83.0 mg, 0.6 mmol), KOH (84.0 mg, 1.5 mmol) and Bu₄NBr (4.0 mg, 0.013 mmol). The mixture was stirred for 5 min at room temperature. Then the appropriate alkyl bromide (for compounds 7-12), benzyl bromide (for compounds 13-15) or ethyl bromoacetate (for compounds 16,17) (0.6 mmol) was added and the mixture was stirred at reflux for 5 h for **7-12, 16-17** and for 2 h for **13-15.** The resulting solution was filtered and the remaining solid material was washed with Et₂O (20 mL). Evaporation of the solvent afforded an oil residue. From the oily crude the different regioisomers were separated by column chromatography eluting with cyclohexane/AcOEt (20:1) to get the isomers **7a-15a** then eluting with cyclohexane/AcOEt (10:1) to separate the isomers **7b-15b** and **7c-15c.** The regioisomers **16-17** were separated by column chromatography eluting with cyclohexane/AcOEt (10:1) to get the isomers **16a-17a** then eluting with cyclohexane/AcOEt (5:1) to obtain the isomer **16b.**

**2-Pentyl-4-*p*-tolyl-2*H*-[1,2,3]triazole (7a):** Colourless oil (71.1 mg, 62%). ¹H NMR (200 MHz, CDCl₃, 25 °C): δ = 7.69 (s, 1 H, H-5), 7.59 (d, ³*J*_{o,m} = 8.0 Hz, 2 H, H*ₒ*), 7.18 (d, ³*J*_{o,m} = 8.0 Hz, 2 H, H*ₘ*), 4.35 (t, ³*J*_{1',*2*'} = 7.0 Hz, 2 H, H-1'), 2.29 (s, 3 H, CH₃), 1.91 (quin, ³*J*_{1',2'} = ³*J*_{2',3'} = 7.0 Hz, 2 H, H-2'), 1.29-1.21 (m, 4 H, H-3', H-4'), 0.82 (t, ³*J*_{4',5'} = 7.0 Hz, 3 H, H-5') ppm; ¹³C NMR (75 MHz, CDCl₃, 25 °C): δ = 147.4 (C-4), 138.0 (C*ₚ*), 130.0 (C-5), 129.4 (C*ₘ*), 127.6 (C*ᵢₚₛₒ*), 125.7 (C*ₒ*), 55.0 (C-1'), 29.5 (C-2'), 28.6 (C-3'), 22.1 (C-4'), 21.2 (CH₃), 13.8 (C-5') ppm. EI-MS: m/z (%) = 229 (100) [M⁺]; 186 (73) [M⁺-43]; 131 (52) [M⁺-98]; 118 (51) [M⁺-111]; 116 (59) [M⁺-113]. C₁₄H₁₉N₃ (229.32): caldc. C 73.33, H 8.35, N 18.32; found C 73.28, H 8.53, N 18.13

**1-Pentyl-4-*p*-tolyl-1*H*-[1,2,3]triazole (7b):** White solid (32.6 mg, 28%); m.p. 73-75 °C (cyclohexane/AcOEt). ¹H NMR (200 MHz, CDCl₃, 25 °C): δ = 7.64 (d, ³*J*_{o,m} = 8.2 Hz, 2 H, H*ₒ*), 7.62 (s, 1 H, H-5), 7.14 (d, ³*J*_{o,m} = 8.2 Hz, 2 H, H*ₘ*), 4.28 (t, ³*J*_{1',2'} = 7.0 Hz, 2 H, H-1'), 2.29 (s, 3 H, CH₃), 1.85 (quin, ³*J*_{1',2'} = ³*J*_{2',3'} = 7.0 Hz, 2 H, H-2'), 1.29-1.18 (m, 4 H, H-3', H-4'), 0.82 (t, ³*J*_{4',5'} = 7.0 Hz, 3 H, H-5') ppm; ¹³C NMR (75 MHz, CDCl₃, 25 °C): δ = 147.7 (C-4), 137.8 (C*ₚ*), 129.4 (C*ₘ*), 127.8 (C*ᵢₚₛₒ*), 125.5 (C*ₒ*), 119.0 (C-5), 50.3 (C-1'), 30.0 (C-2'), 28.5 (C-3'), 22.0 (C-4'), 21.2 (CH₃), 13.8 (C-5') ppm. EI-MS: m/z (%) = 229 (50) [M⁺]; 159 (43) [M⁺-70]; 131 (100) [M⁺-98]. C₁₄H₁₉N₃ (229.32): caldc. C 73.33, H 8.35, N 18.32; found C 73.48, H 8.47, N 18.01

**1-Pentyl-5-*p*-tolyl-1*H*-[1,2,3]triazole (7c):** White solid (5.7 mg, 5%); m.p. 102-104 °C (cyclohexane/AcOEt). ¹H NMR (500 MHz, CDCl₃, 25 °C): δ = 7.56 (s, 1 H, H-4), 7.19-7.16 (m, 4 H, H*ₒ*, H*ₘ*), 4.26 (t, *³J*_{1',2'} = 7.0 Hz, 2 H, H-1'), 2.33 (s, 3 H, CH₃), 1.73 (quin, ³*J*_{1',2'} = *³J*_{2',3'} = 7.0 Hz, 2 H, H-2'), 1.27-1.15 (m, 4 H, H-3', H-4'), 0.74 (t, ³*J*_{4',5'} = 7.0 Hz, 3 H, H-5') ppm; ¹³C NMR (75 MHz, CDCl₃, 25 °C): δ = 139.5 (C*ₚ*), 137.7 (C-5), 132.9 (C-4), 129.7 (C*ₘ*), 128.6 (C*ₒ*), 124.3 (C*ᵢₚₛₒ*), 48.2 (C-1'), 29.8 (C-2'), 28.5 (C-3'), 22.0 (C-4'), 21.3 (CH₃), 13.8 (C-5') ppm. EI-MS: m/z (%) = 229 (16) [M⁺]; 131 (100) [M⁺-98]; 116 (67) [M⁺-113]. C₁₄H₁₉N₃ (229.32): caldc. C 73.33, H 8.35, N 18.32; found C 73.80, H 8.25, N 18.01

**4-(*p*-Methoxyphenyl)-2-pentyl-2*H*[1,2,3]triazole (8a):** Yellow solid (55.2 mg, 45%); m.p. 52-54 °C (cycloheXane/AcOEt). ¹H NMR (200 MHz, CDCl₃, 25 °C): δ = 7.75 (s, 1 H, H-5), 7.71 (d, ³*J*_{o,m} = 9.0 Hz, 2 H, H*ₒ*), 6.96 (d, ³*J*_{o,m} = 9.0 Hz, 2 H, H*ₘ*), 4.43 (t, ³*J*_{1',2}, = 7.0 Hz, 2 H, H-1'), 3.85 (s, 3 H, CH₃), 2.00 (quin, ³*J*_{1',2'} = ³*J*_{2',3'} = 7.0 Hz, 2 H, H-2'), 1.38-1.26 (m, 4 H, H-3', H-4'), 0.91 (t, ³*J*_{4',5'} = 7.0 Hz, 3 H, H-5') ppm; ¹³C NMR (50.5 MHz, CDCl₃, 25 °C): δ = 159.7 (C*ₚ*), 147.3 (C-4), 130.0 (C-5), 127.1 (C*ₒ*), 123.3 (C*ᵢₚₛₒ*), 114.2 (C*ₘ*), 55.3 (CH₃), 55.0 (C-1'), 29.5 (C-2'), 28.6 (C-3'), 22.1 (C-4'), 13.9 (C-5') ppm. EI-MS: m/z (%) = 245 (100) [M⁺]; 202 (52) [M⁺-43]; 133 (54) [M⁺-112]; 132 (88) [M⁺-113]. C₁₄H₁₉N₃O (245.32): caldc. C 68.54, H 7.81, N 17.13; found C 68.65, H 8.12, N 16.97

**4-(*p*-Methoxyphenyl)-1-pentyl-1*H*-[1,2,3]triazole (8b):** Yellow solid (30.7 mg, 25%); m.p. 100-102 °C (cyclohexane/AcOEt). ¹H NMR (200 MHz, CDCl₃, 25 °C): δ = 7.76 (d, ³*J*_{o,m}, = 8.7 Hz, 2 H, H*ₒ*), 7.67 (s, 1 H, H-5), 6.96 (d, ³*J*_{o,m} = 8.7 Hz, 2 H, H*ₘ*), 4.38 (t, *³J*_{1',2'} = 7.2 Hz, 2 H, H-1'), 3.85 (s, 3 H, CH₃), 1.95 (quin, ³*J*_{1',2'} = ³*J*_{2',3'} = 7.2 Hz, 2 H, H-2'), 1.39-1.33 (m, 4 H, H-3', H-4'), 0.91 (t, ³*J*_{4',5'} = 6.7 Hz, 3 H, H-5') ppm; ¹³C NMR (50.5 MHz, CDCl₃, 25 °C): δ = 159.5 (C*ₚ*), 147.5 (C-4), 126.9 (C*ₒ*), 123.5 (C*ᵢₚₛₒ*), 118.6 (C-5), 114.2 (C*ₘ*), 56.3 (CH₃), 50.0 (C-1'), 30.0 (C-2'), 28.6 (C-3'), 22.1 (C-4'), 13.8 (C-5') ppm. EI-MS: m/z (%) = 245 (76) [M⁺]; 175 (93) [M⁺-70]; 147 (100) [M⁺-98]; 132 (79) [M⁺-113]. C₁₄H₁₉N₃O (245.32): caldc. C 68.54, H 7.81, N 17.13; found C 68.42, H 7.97, N 17.80

**4-(4-Bromo-2-fluorophenyl)-2-pentyl-2*H*-[1,2,3]triazole (9a):** Colourless oil (107.7 mg, 69%). ¹H NMR (200 MHz, CDCl₃, 25 °C): δ = 7.84 (d, ⁵*J*_{5,F} = 3.8 Hz, 1 H, H-5), 7.80 (dd, ³*J*_{5",6"} = 8.5 Hz, ⁴*J*_{6",F} = 8.0 Hz, 1 H, H-6"), 7.29-7.18 (m, 2 H, H-3", H-5"), 4.37 (t, ³*J*_{1',2'} = 7.0 Hz, 2 H, H-1'), 1.92 (quin, ³*J*_{1',2'} = ³*J*_{2',3'} = 7.0 Hz, 2 H, H-2'), 1.34-1.31 (m, 4 H, H-3', H-4'), 0.82 (t, *³J*_{4,5} *=* 6.7 Hz, 3 H, H-5') ppm; ¹³C NMR (50.5 MHz, CDCl₃, 25 °C): δ = 159.4 (d, ¹*J*_{2",F} = 254.7 Hz, C-2"), 140.9 (C-4), 133.4 (d, ⁴*J*_{5,F} = 12.5 Hz, C-5), 129.1 (d, ³*J*_{6",F} = 4.4 Hz, C-6"), 127.8 (d, ⁴*J*_{5",F} = 3.5 Hz, C-5"), 121.8 (d, ³*J*_{4",F} = 9.9 Hz, C-4"), 119.6 (d, ²*J*_{3",F} = 25.1 Hz, C-3"), 117.7 (d, ²*J*_{1",F} = 12.6 Hz, C-1"), 55.1 (C-1'), 29.4 (C-2'), 28.6 (C-3'), 22.1 (C-4'), 13.9 (C-5') ppm. EI-MS: m/z (%) = 313, 311 (100) [M⁺]; 285, 283 (72) [M⁺-28]; 270, 268 (89) [M⁺-43]; 256, 254 (56) [M⁺-57]; 244, 242 (56) [M⁺-71]; 243, 241 (57) [M⁺-70]; 215, 213 (70) [M⁺-98]; 202, 200 (55) [M⁺-111]; 200, 198 (49) [M⁺-113]. C₁₃H₁₅BrFN₃ (312.18): caldc. C 50.02, H 4.84, N 13.46; found C 50.68, H 4.48 N 13.93

**4-(4-Bromo-2-fluorophenyl)-1-pentyl-1*H*-[1,2,3]triazole (9b):** Yellow solid (45.3 mg, 29%); m.p. 71-73 °C (cyclohexane/AcOEt). ¹H NMR (200 MHz, CDCl₃, 25 °C): δ = 8.12 (t, ³*J*_{5",6"} = ⁴*J*_{6",F} = 8.2 Hz, 1 H, H-6"), 7.82 (d, ⁵*J*_{5-F} = 3.8 Hz, 1 H, H-5), 7.35-7.15 (m, 2 H, H-3", H-5"), 4.33 (t, ³*J*_{1',2'} = 7.0 Hz, 2 H, H-1'), 1.89 (quin, ³*J*_{1',2'} = ³*J*_{2',3'} = 7.0 Hz, 2 H, H-2'), 1.33-1.26 (m, 4 H, H-3', H-4'), 0.84 (t, ³*J*_{4',5'} = 6.9 Hz, 3 H, H-5') ppm; ¹³C NMR (50.5 MHz, CDCl₃, 25 °C): δ = 158.7 (d, ¹*J*_{2",F} = 258.7 Hz, C-2"), 140.2 (d, ³*J*_{4,F} = 3.1 Hz, C-4), 128.7 (d, ³*J*_{6",F} = 6.1 Hz, C-6"), 128.0 (d, ⁴*J*_{5",F} = 3.9 Hz, C-5"), 122.5 (d, ⁴*J*_{5,F} = 13.9 Hz, C-5), 121.4 (d, ³*J*_{4",F} = 8.9 Hz, C-4"), 119.2 (d, ²*J*_{3",F} = 25.1 Hz, C-3"), 117.9 (d, ²*J*_{1",F} = 13.0 Hz, C-1"), 50.4 (C-1'), 29.9 (C-2'), 28.5 (C-3'), 22.0 (C-4'), 13.8 (C-5') ppm. EI-MS: m/z (%) = 313, 311 (71) [M⁺]; 243, 241 (52) [M⁺-70]; 215, 213 (100) [M⁺-98]; 202, 200 (66) [M⁺-111]. C₁₃H₁₅BrFN₃ (312.18): caldc. C 50.02, H 4.84, N 13.46; found C 50.19, H 4.96, N 13.17

**4-(*p*-Chlorophenyl)-2-pentyl-5-phenyl-2*H*-[1,2,3]triazole (10a):** Colourless oil (81.5 mg, 50%). ¹H NMR (200 MHz, CDCl₃, 25 °C): δ = 7.46-7.37 (m, 4 H, H*ₒ*, H*ₒ*), 7.30-7.20 (m, 5 H, H*_{m'},* H*_{p'},* H*ₘ*), 4.36 (t, ³*J*_{1',2'} = 7.0 Hz, 2 H, H-1'), 1.95 (quin, ³*J*_{1',2'} = ³*J*_{2',3'} = 7.0 Hz, 2 H, H-2'), 1.32-1.25 (m, 4 H, H-3', H-4'), 0.83 (t, ³*J*_{4',5'} = 7.0 Hz, 3 H, H-5') ppm; ¹³C NMR (75 MHz, CDCl₃, 25 °C): δ = 144.2 (C-5), 142.9 (C-4), 134.1 (C*ₚ*), 130.9 (C*_{ipso'}*), 130.0 (C*ᵢₚₛₒ*), 129.4 (C*ₒ*), 128.7 (C*ₘ*), 128.6 (C*_{m'}*), 128.3 (C*_{p'}*), 128.2 (C*_{o'}*), 55.6 (C-1'), 29.9 (C-2'), 29.1 (C-3'), 22.6 (C-4'), 14.6 (C-5') ppm. EI-MS: m/z (%) = 327 (42), 325 (100) [M⁺]; 296 (58) [M⁺-29]; 282 (51) [M⁺-43]; 227 (60) [M⁺-98]; 165 (56) [M⁺-160]. C₁₉H₂₀ClN₃ (325.84): caldc. C 70.04, H 6.19, N 12.90; found C 70.44, H 6.10, N 12.76

**4-(*p*-Chlorophenyl)-1-pentyl-5-phenyl-1*H*-[1,2,3]triazole (10b):** White solid (50.5 mg, 31%); m.p. 85-87 °C (cyclohexane/AcOEt). ¹H NMR (200 MHz, CDCl₃, 25 °C): δ = 7.54-7.48 (m, 3 H, H*_{m'}*, H*_{p'}*), 7.44 (dt, ³*J*_{o,m} = 8.6 Hz, ⁴*J*_{o,o} = ⁵*J*_{o,m} = 2.2 Hz, 2 H, H*ₒ*), 7.32-7.26 (m, 2 H, H*_{o'}*), 7.19 (dt, ³*J*_{o,m} = 8.6 Hz, ⁴*J*_{m,m} = ⁵*J*_{o,m} = 2.2 Hz, 2 H, H*ₘ*), 4.17 (t, ³*J*_{1',2'} = 7.3 Hz, 2 H, H-1'), 1.76 (quin, ³*J*_{1',2'}=³*J*_{2',3'} = 7.3 Hz, 2 H, H-2'), 1.24-1.12 (m, 4 H, H-3', H-4'), 0.80 (t, ³*J*_{4',5'} = 6.5 Hz, 3 H, H-5') ppm; ¹³C NMR (75 MHz, CDCl₃, 25 °C): δ = 143.1 (C-4), 133.7 (C-5), 133.3 (C*ₚ*), 129.8 (C*_{p'}*, C*_{o'}*), 129.5 (C*ᵢₚₛₒ*), 129.4 (C*_{m'}*), 128.6 (C*ₘ*), 127.9 (C*ₒ*, *C_{ipso'}*), 48.2 (C-1'), 29.7 (C-2'), 28.4 (C-3'), 21.9 (C-4'), 13.7 (C-5') ppm. EI-MS: m/z (%) = 327 (12), 325 (34) [M⁺]; 229 (34), 227 (100) [M⁺-98]; 228 (44), 226 (90) [M⁺-99]; 165 (46) [M⁺-160]. C₁₉H₂₀ClN₃ (325.84): caldc. C 70.04, H 6.19, N 19.20; found C 69.94, H 6.18, N 19.25

**5-(*p*-Chlorophenyl)-1-pentyl-4-phenyl-1*H*-[1,2,3]triazole (10c):** White solid (14.7 mg, 9%); m.p. 110-112 °C (cyclohexane/AcOEt). ¹H NMR (300 MHz, CDCl₃, 25 °C): δ = 7.52-7.47 (m, 4 H, H*ₒ*, H*_{m'}*), 7.29-7.24 (m, 5 H, *H_{o'},* H*ₚ,* H*ₘ*), 4.18 (t, ³*J*_{1',2'} = 7.4 Hz, 2 H, H-1'), 1.78 (2H, quin, ³*J*_{1'.2'} = ³*J*_{2',3'} = 7.4 Hz, H-2'), 1.33-1.20 (4H, m, H-3', H-4'), 0.83 (3H, t, ³*J*_{4',5'} = 6.8 Hz, H-5') ppm; ¹³C NMR (75 MHz, CDCl₃, 25 °C): δ = 144.4 (C-4), 135.9 (C*_{p'}*), 132.4 (C-5), 131.3 (C*_{o'}*), 130.7 (C*ᵢₚₛₒ*), 129.7 (C*_{m'}*), 128.5 (C*ₘ*), 127.8 (C*ₚ*), 126.8 (C*ₒ*), 126.7 (C*_{ipso'}*), 48.3 (C-1'), 29.7 (C-2'), 28.5 (C-3'), 22.0 (C-4'), 13.8 (C-5') ppm. EI-MS: m/z (%) = 327 (10), 325 (27) [M⁺]; 229 (34), 227 (100) [M⁺-98]; 228 (36), 226 (67) [M⁺-99]; 165 (45) [M⁺-160]. C₁₉H₂₀ClN₃ (325.84): caldc. C 70.04, H 6.19, N 19.20; found C 70.29, H 6.05, N 19.35

**4-(2,4-Dichlorophenyl)-2-pentyl-5-*p*-tolyl-2*H*-[1,2,3]triazole (11a):** Colourless oil (136.6 mg, 73%). ¹H NMR (300 MHz, CDCl₃, 25 °C): δ = 7.43 (d, ⁴*J*_{3",5"} = 2.4 Hz, 1 H, H-3"), 7.39 (d, ³*J*_{5",6"} = 8.4 Hz, 1 H, H-6"), 7.33-7.32 (m, 3 H, H-5", H*ₒ*), 7.11 (d, ³*J*_{o,m} = 8.0 Hz, 2 H, H*ₘ*), 4.47 (t, ³*J*_{1',2'} = 7.0 Hz, 2 H, H-1'), 2.32 (s, 3 H, CH₃), 2.05 (quin, ³*J*_{1',2'} = ³*J*_{2',3'} = 7.0 Hz, 2 H, H-2'), 1.38-1.35 (m, 4 H, H-3', H-4'), 0.91 (t, ³*J*_{4',5'} = 7.0 Hz, 3 H, H-5') ppm; ¹³C NMR (75 MHz, CDCl₃, 25 °C): δ = 145.2 (C-5), 140.4 (C-4), 138.1 (C*ₚ*), 132.8 (C-1"), 132.7 (C-2"), 132.6 (C-4"), 131.8 (C-3"), 131.0 (C6"), 130.1 (C5"), 129.3 (C*ₘ*), 127.8 (C*ᵢₚₛₒ*), 126.6 (C*ₒ*), 55.3 (C-1'), 29.5 (C-2'), 28.7 (C-3'), 22.1 (C-4'), 21.3 (CH₃), 13.9 (C-5') ppm. EI-MS: m/z (%) = 377 (16), 375 (78), 373 (100) [M⁺]; 330 (59) [M⁺-43]; 275 (62) [M⁺-98]; 213 (43) [M⁺-161]; 118 (68) [M⁺-255]. C₂₀H₂₁Cl₂N₃ (374.31): caldc. C 64.18, H 5.65, N 11.23; found C 64.21, H 5.46, N 11.08

**4-(*p*-Chlorophenyl)-2-heptyl-5-phenyl-2*H*-[1,2,3]triazole (12a):** Colourless oil (113.2 mg, 64%). ¹H NMR (200 MHz, CDCl₃, 25 °C): δ = 7.52-7.46 (m, 4 H, H*ₒ*, H_{*o*'}), 7.37-7.30 (m, 5 H, H_{*m*',} H*ₚ*, H*ₘ*), 4.44 (t, ³*J*_{1',2'} = 7.2 Hz, 2 H, H-1'), 2.02 (quin, ³*J*_{1',2'} = ³*J*_{2',3'} = 7.2 Hz, 2 H, H-2'), 1.39-1.26 (m, 8 H, H-3', H-4', H-5', H-6'), 0.86 (t, 3 H, ³*J*_{6',7'} = 7.2 Hz, H-7') ppm; ¹³C NMR (75 MHz, CDCl₃, 25 °C): δ = 144.2 (C-5), 142.9 (C-4), 134.1 (C*ₚ*), 130.9 (C_{*ipso*'}), 129.7 (C*ᵢₚₛₒ*), 129.4 (C*ₒ*), 128.8 (C*ₘ*), 128.6 (C_{*m*'}), 128.4 (C_{*p*'}), 128.2 (C_{*o*'}), 55.2 (C-1'), 31.6 (C-5'), 29.8 (C-2'), 28.7 (C-4'), 26.5 (C-3'), 22.4 (C-6'), 14.0 (C-7') ppm. EI-MS: m/z (%) = 355 (35), 353 (97) [M⁺]; 282 (87) [M⁺-71]; 256 (50) [M⁺-98]; 229 (35), 227 (100) [M⁺-128]; 165 (79) [M⁺-188]. C₂₁H₂₄ClN₃ (353.89): caldc. C 71.27, H 6.84, N 11.87; found C 71.63, H 6.97, N 11.68

**4-(*p*-Chlorophenyl)-1-heptyl-5-phenyl-1*H*-[1,2,3]triazole (12b):** White solid (31.8 mg, 18%); m.p. 64-66 °C (cyclohexane/AcOEt). ¹H NMR (300 MHz, CDCl₃, 25 °C): δ = 7.47-7.44 (m, 3 H, H*_{m'}*, H*_{p'}*), 7.40 (dt, ³*J*_{o,m} = 8.4 Hz, ⁴*J*_{o,o} = ⁵*J*_{o,m} = 2.2 Hz, 2 H, H*ₒ*), 7.25-7.22 (m, 2 H, H*_{o'}*), 7.16 (dt, ³*J*_{o,m} = 8.4 Hz, ⁴*J*_{m,m} = ⁵*J*_{o,m} = 2.2 Hz, 2 H, H*ₘ*), 4.12 (t, ³*J*_{1',2'} = 7.2 Hz, 2 H, H-1'), 1.17 (quin, ³*J*_{1',2'} =³*J*_{2',3'} = 7.2 Hz, 2 H, H-2'), 1.18-1.13 (m, 8 H, H-3', H-4', H-5', H-6'), 0.78 (t, 3 H, ³*J*_{6',7'} = 7.2 Hz, H-7') ppm; ¹³C NMR (75 MHz, CDCl₃, 25 °C): δ = 143.1 (C-4), 133.8 (C-5), 133.4 (C*ₚ*), 129.9 (C*_{o'}*), 129.8 (C*_{p'}*), 129.6 (C*ᵢₚₛₒ*), 129.4 (C*_{m'}*), 128.6 (C*ₘ*), 127.9 (C*ₒ*, C*_{ipso'}*), 48.3 (C-1'), 31.4 (C-5'), 30.0 (C-2'), 28.5 (C-4'), 26.3 (C-3'), 22.4 (C-6'), 14.0 (C-7') ppm. EI-MS: m/z (%) = 355 (10), 353 (28) [M⁺]; 229 (35), 227 (100) [M⁺-128]; 228 (45), 226 (86) [M⁺-129]. C₂₁H₂₄ClN₃ (353.89): caldc. C 71.27, H 6.84, N 11.87; found C 71.32, H 7.91, N 11.80

**5-(*p*-Chlorophenyl)-1-heptyl-4-phenyl-1*H*-[1,2,3]triazole (12c):** Colourless oil (15.9 mg, 9%). ¹H NMR (300 MHz, CDCl₃, 25 °C): δ = 7.46-7.41 (m, 4 H, H*ₒ*, H_{*m*'}), 7.21-7.17 (m, 5 H, H_{*o*',} H*ₚ,* H*ₘ*), 4.12 (t, ³*J*_{1',2'} = 7.2 Hz, 2 H, H-1'), 1.71 (quin, ³*J*_{1',2'} = ³*J*_{2',3'} = 7.2 Hz, 2 H, H-2'), 1.18-1.14 (m, 8 H, H-3', H-4', H-5', H-6'), 0.77 (t, 3 H, ³*J*_{6',7'} = 7.2 Hz, H-7') ppm; ¹³C NMR (75 MHz, CDCl₃, 25 °C): δ = 144.3 (C-4), 135.9 (C_{*p*'}), 132.5 (C-5), 131.3 (C_{*o*'}), 130.7 *(Cᵢₚₛₒ),* 129.8 (C_{*m*'}), 128.5 (C*ₘ*), 127.8 (C*ₚ*), 126.9 (C*ₒ*), 126.8 (C*_{ipso'}*), 48.3 (C-1'), 31.4 (C-5'), 30.0 (C-2'), 28.5 (C-4'), 26.4 (C-3'), 22.4 (C-6'), 14.0 (C-7') ppm. EI-MS: m/z (%) = 355 (7), 353 (19) [M⁺]; 229 (35), 227 (100) [M⁺-128]; 228 (38), 226 (65) [M⁺-129]. C₂₁H₂₄ClN₃ (353.89): caldc. C 71.27, H 6.84, N 11.87; found C 71.40, H 6.80, N 11.98

**2-(*p*-Bromobenzyl)-4-(*p*-chlorophenyl)-5-phenyl-2*H*-[1,2,3]triazole (13a):** White solid (131.6 mg, 62%); m.p. 104-106 °C (cyclohexane). ¹H NMR (300 MHz, CDCl₃, 25 °C): δ = 7.52-7.45 (m, 6 H, H*ₒ*, H*_{o'}*, H*_{m"}*), 7.38-7.34 (m, 3 H, H*_{m'}*, H*ₚ*), 7.33-7.29 (m, 4 H, H*ₘ*, H*_{o"}*), 5.57 (s, 2 H, CH₂) ppm; ¹³C NMR (75 MHz, CDCl₃, 25 °C): δ = 145.1 (C-5), 143.9 (C-4), 134.3 (C*_{ipso"}*), 134.0 (C*ₚ*), 131.9 (C*_{m"}*), 130.5 (C*_{ipso'}*), 129.9 (C*_{o"}*), 129.5 (C*ₒ*), 129.3 (C*ᵢₚₛₒ*), 128.8 (C*ₘ*), 128.7 (C*_{m'}*), 128.6 (C*_{p'}*), 128.2 (C*_{o'}*), 122.5 (C*_{p"}*), 58.1 (CH₂) ppm. EI-MS: m/z (%) = 427 (36), 425 (97), 423 (78) [M⁺]; 228 (47), 226 (100) [M⁺-199]; 171 (82), 169 (80) [M⁺-255]. C₂₁H₁₅ClBrN₃ (424.72): caldc. C 59.39, H 3.56, N 9.89; found C 59.10, H 3.72, N 9.38

**1-(*p*-Bromobenzyl)-4-(*p*-chlorophenyl)-5-phenyl-1*H*-[1,2,3]triazole (13b):** White solid (29.7 mg, 14%); m.p. 120-122 °C (cyclohexane). ¹H NMR (300 MHz, CDCl₃, 25 °C): δ = 7.49-7.44 (m, 5 H, H*_{m'}*, H*_{m"}*, H*_{p'}*), 7.3 8 (dt, ³*J*_{o,m} = 8.4 Hz, ⁴*J*_{o,o} = ⁵*J*_{o,m} = 2.4 Hz, 2 H, H*ₒ*), 7.22 (dt, ³*J*_{o,m} = 8.4 Hz, ⁴*J*_{m,m} *=* ⁵*J*_{o,m} = 2.4 Hz, 2 H, H*ₘ*), 7.15-7.11 (m, 2 H, H*_{o'}*), 6.89 (d, ³*J*_{o",m"} = 8.4 Hz, 2 H, H*_{o"}*), 5.34 (s, 2 H, CH₂) ppm; ¹³C NMR (75 MHz, CDCl₃, 25 °C): δ = 144.1 (C-4), 134.1 (C*_{ipso"}*), 133.9 (C-5), 133.6 (C*ₚ*), 131.9 (C*_{m"}*), 130.0 (C*_{p'}*), 129.9 (*C_{o'},* C*ᵢₚₛₒ*), 129.4 (C*_{m'}*), 129.2 (C*_{o"}*), 128.7 (C*ₘ*), 127.9 (C*ₒ*), 127.4 (C*_{ipso'}*), 122.4 (C*_{p"}*), 51.8 (CH₂) ppm. EI-MS: m/z (%) = 425 (6) [M⁺]; 228 (37), 226 (100) [M⁺-199]; 171 (52), 169 (54) [M⁺-255]. C₂₁H₁₅ClBrN₃ (424.72): caldc. C 59.39, H 3.56, N 9.89; found C 59.42, H 3.91, N 9.53

**1-(*p*-Bromobenzyl)-5-(*p*-chlorophenyl)-4-phenyl-1*H*-[1,2,3]triazole (13c):** White solid (40.3 mg, 19%); m.p. 164-166 °C (cyclohexane). ¹H NMR (300 MHz, CDCl₃, 25 °C): δ = 7.54-7.50 (m, 2 H, H*_{m''}*), 7.43-7.39 (m, 4 H, H*ₒ*, H*_{m'}*) 7.31-7.26 (m, 3 H, H*ₘ*, H*ₚ*), 7.06 (dt, ³*J*_{o',m'} = 8.6 Hz, ⁴*J*_{o',o'} = ⁵*J*_{o',m'} = 2.0 Hz, 2 H, H*_{o'}*) 6.92 (d, ³*J*_{o",m"} = 8.5 Hz, 2 H, H_{*o*"}), 5.35 (s, 2 H, CH₂) ppm; ¹³C NMR (75 MHz, CDCl₃, 25 °C): δ = 145.0 (C-4), 136.3 (C*_{p'}*), 134.1 (C-5), 134.0 (C*_{ipso"}*), 131.9 (C*_{m"}*), 131.4 (C*_{o'}*), 130.4 (C*ᵢₚₛₒ*), 129.7 (C*ₘ*), 129.1 (C*_{o"}*), 128.6 (C*ₘ*), 128.0 (C*ₚ*), 126.7 (C*ₒ*), 126.1 (C*_{ipso'}*), 122.3 (C*_{p"}*), 58.1 (CH₂) ppm. EI-MS: m/z (%) = 425 (10) [M⁺]; 228 (39), 226 (100) [M⁺-199]; 171 (49), 169 (51) [M⁺-255]. C₂₁H₁₅ClBrN₃ (424.72): caldc. C 59.39, H 3.56, N 9.89; found C 59.22, H 3.88, N9.77

**2-Benzyl-4-(*p*-chlorophenyl)-5-phenyl-2*H*-[1,2,3]triazole (14a):** White solid (110.7 mg, 64%); m.p. 110-112 °C (cyclohexane). ¹H NMR (500 MHz, CDCl₃, 25 °C): δ = 7.52-7.45 (m, 4 H, H*ₒ*, H_{*o*'}), 7.46-7.42 (m, 2 H, H_{*o*"}), 7.40-7.32 (m, 8 H, H_{*m*"}, H_{*p*"}, H_{*m*'}, H_{*p*'}, H*ₘ*), 5.62 (s, 2 H, CH₂) ppm; ¹³C NMR (75 MHz, CDCl₃, 25 °C): δ = 145.1 (C-5), 143.9 (C-4), 135.4 (C_{*ipso*"}), 134.5 (C*ₚ*), 131.0 (C_{*ipso*'}), 129.8 (C*ᵢₚₛₒ*, C*ₒ*), 129.1 (C_{*o*"}), 129.0 (C*ₘ*), 128.9 (C_{*m*'}), 128.7 (C_{*p*"}), 128.6 (C_{*p*'}), 128.5 (C_{*o*'}) 128.4 (C_{*m*"}), 59.0 (CH₂) ppm. EI-MS: m/z (%) = 347 (58), 345 (100) [M⁺]; 226 (79) [M⁺-119]; 91 (99) [M⁺-254]. C₂₁H₁₆ClN₃ (345.82): caldc. C 72.93, H 4.66, N 12.15; found C 72.68, H 4.75, N 12.10

**1-Benzyl-4-(*p*-chlorophenyl)-5-phenyl-1*H*-[1,2,3]triazole (14b):** White solid (41.4 mg, 24%); m.p. 145-147 °C (cyclohexane). ¹H NMR (500 MHz, CDCl₃, 25 °C): δ = 7.51-7.46 (m, 3 H, H_{*m*',} H_{*p*'}), 7.44-7.40 (m, 2 H, H*ₒ*), 7.26-7.20 (m, 5 H, H_{*m*",} H_{*p*",} H_{*m*'}), 7.14-7.10 (m, 2 H, H*ₒ*), 7.03-7.00 (m, 2 H, H_{*o*"}), 5.40 (s, 2 H, CH₂) ppm; ¹³C NMR (125 MHz, CDCl₃, 25 °C): δ = 143.5 (C-4), 135.2 (C_{*ipso*"}), 134.0 (C-5), 133.1 *(*C*_{p"})*, 130.0 (C*ᵢₚₛₒ*, C_{*o*'}), 129.8 (C_{*p*'}), 129.4 (C_{*p*"}), 129.3 (C_{*m*'}), 128.6 (C*ₘ*, C_{*m*"}), 128.2 (C_{*p*"}), 127.9 (C_{*ipso*'}), 127.5 (C_{*o*"}), 52.1 (CH₂) ppm. EI-MS: m/z (%) = 347 (20), 345 (51) [M⁺]; 228 (58), 226 (100) [M⁺-119]; 91 (96) [M⁺-255]. C₂₁H₁₆ClN₃ (345.82): caldc. C 72.93, H 4.66, N 12.15; found C 72.82, H 4.90, N 11.93

**1-Benzyl-5-(*p*-chlorophenyl)-4-phenyl-1*H*-[1,2,3]triazole (14c):** White solid (15.5 mg, 9%); m.p. 177-179 °C (cyclohexane). ¹H NMR (200 MHz, CDCl₃, 25 °C): δ = 7.57-7.52 (m, 2 H, H*ₒ*), 7.43-7.39 (d, ³*J*_{m',o'} = 8.4 Hz, 2 H, H_{*m*'}), 7.30-7.28 (m, 6 H, H_{*o*"}, H_{*p*"}, H*ₘ*, H*ₚ*), 7.10-7.06 (m, 4 H, H_{*o*'}, H_{*m*"}), 5.43 (s, 2 H, CH₂) ppm; ¹³C NMR (125 MHz, CDCl₃, 25 °C): δ = 144.8 (C-4), 136.0 (C_{*p*'}), 135.2 (C_{*ipso*"}), 132.7 (C-5), 131.4 (C_{*o*'}), 130.6 (C*ᵢₚₛₒ*), 129.5 (C_{*m*'}), 128.8 (C_{*m*"}), 128.5 (C*ₘ*), 127.9 (C*ₚ*), 127.3 (C_{*p*"}), 127.3 (C_{*o*"}), 126.7 (C*ₒ*), 126.3 (C*ᵢₚₛₒ*), 52.1 (CH₂) ppm. EI-MS: m/z (%) = 347 (14), 345 (36) [M⁺]; 228 (43), 226 (95) [M⁺-119]; 91 (100) [M⁺-255]. C₂₁H₁₆ClN₃ (345.82): caldc. C 72.93, H 4.66, N 12.15; found C 72.98, H 5.04, N 11.63

**2-Benzyl-4-(4-bromo-2-fluorophenyl)-2*H*-[1,2,3]triazole (15a):** White solid (73.1 mg, 44%); m.p. 104-106 °C (cyclohexane). ¹H NMR (500 MHz, CDCl₃, 25 °C): δ = 7.97 (d, ⁵*J*_{5,F} = 3.9 Hz, 1 H, H-5), 7.88 (t, ³*J*_{5',6'} = ⁴*J*_{6',F} = 8.3 Hz, 1 H, H-6'), 7.40-7.34 (m, 7 H, H*ₒ*, H*ₘ*, H*ₚ*, H-3', H-5'), 5.60 (s, 2 H, CH₂) ppm; ¹³C NMR (50 MHz, CDCl₃, 25 °C): δ = 159.8 (d, ¹*J*_{2',F} = 254.1 Hz, C-2'), 141.9 (d, ³*J*_{4,F} = 1.5 Hz, C-4), 135.3 (C*ᵢₚₛₒ*), 134.4 (d, ⁴*J*_{5,F} = 11.4 Hz, C-5), 129.5 (d, ³*J*_{6',F} = 4.4 Hz, C-6'), 129.1 (C*ₒ*) 128.7 (C*ₚ*), 128.3 (C*ₘ*), 128.1 (d, ⁴*J*_{5',F} = 3.8 Hz, C-5'), 122.3 (d, ³*J*_{4',F} = 9.9 Hz, C-4'), 119.9 (d, ²*J*_{3',F} = 25.2 Hz, C-3'), 117.8 (d, ²*J*_{1',F} = 13.0 Hz, C-1'), 59.1 (CH₂) ppm. EI-MS: m/z (%) = 333 (83), 331 (80) [M⁺]; 214 (44), 212 (43) [M⁺-119]; 91 (100) [M⁺-241]. C₁₅H₁₁BrFN₃ (332.17): caldc. C 54.24, H 3.34, N 12.65; found C 53.98, H 3.21, N 12.42

**1-Benzyl-4-(4-bromo-2-fluorophenyl)-1*H*[1,2,3]triazole (15b):** White solid (89.7 mg, 54%); m.p. 146-148 °C (cyclohexane). ¹H NMR (500 MHz, CDCl₃, 25 °C): δ = 8.17 (dd, ³*J*_{6',5'} = 8.3 Hz, ⁴*J*_{6',F} = 7.8 Hz, 1 H, H-6'), 7.83 (d, ⁵*J*_{5,F} = 3.4 Hz, 1 H, H-5), 7.39-7.34 (m, 4 H, H-5', H*ₘ*, H*ₚ*), 7.30-7.24 (m, 3 H, H-3', H*ₒ*), 5.57 (s, 2 H, CH₂) ppm; ¹³C NMR (50 MHz, CDCl₃, 25 °C): δ =158.7 (d, ¹*J*_{2',F} =251.7 Hz, C-2'), 140.6 (d, *³J*_{4.F} = 3.1 Hz, C-4), 134.5 (C*ᵢₚₛₒ*), 129.1 (C*ₘ*), 128.8 (C*ₚ*), 128.7 (d, ³*J*_{6',F} = 4.6 Hz, C-6'), 127.9 (C*ₒ*, C-5'), 122.6 (d, ⁴*J*_{5,F} = 12.2 Hz, C-5), 121.6 (d, ³*J*_{4',F} = 11.0 Hz, C-4'), 119.2 (d, ²*J*_{3',F} = 25.2 Hz, C-3'), 117.8 (d, ²*J*_{1',F} = 12.9 Hz, C-1'), 54.2 (CH₂) ppm. EI-MS: m/z (%) = 333 (35), 331 (34) [M⁺]; 304 (50), 302 (49) [M⁺-29]; 214 (75), 212 (73) [M⁺-119]; 197 (50) [M⁺-137]; 91 (100) [M⁺-241]. C₁₅H₁₁BrFN₃ (332.17): caldc. C 54.24, H 3.34, N 12.65; found 54.15, H 3.45, N 12.38

**4-(4-Bromo-2-fluorophenyl)-2-(ethoxycarbonyl)methyl-2*H*[1,2,3]-triazole (16a):** White solid (90.2, 55%); m.p. 107-109 °C (cyclohexane/Et₂O). ¹H NMR (200 MHz, CDCl₃, 25 °C): δ = 8.04 (d, ⁵*j*_{5,F} = 4.0 Hz, 1 H, H-5), 7.89 (dd, ³*J*_{6',5'} = 8.5 Hz, ⁴*J*_{6',F} = 7.2 Hz, 1 H, H-6'), 7.37 (d, ³*J*_{5',6'} *=* 8.5 Hz, ³*J*_{3',F} *=* 8.5 Hz, 2 H, H-5', H-3'), 5.27 (s, 2 H, CH₂), 4.28 (q, ³*J*_{1",2"} = 7.0 Hz, 2 H, H-1"), 1.30 (t, ³*J*_{1",2"} = 7.0 Hz, 3 H, H-2") ppm. ¹³C NMR (50 MHz, CDCl₃, 25 °C): δ = 166.5 (CO), 159.5 (d, ¹*J*_{2',F} = 254.7 Hz, C-2'), 142.3 (d, ³*J*_{4,F} = 2.3 Hz, C-4), 134.7 (d, ⁴*J*_{5,F} = 12.4 Hz, C-5), 129.3 (d, ³*J*_{6',F} = 4.6 Hz, C-6'), 127.9 (d, ⁴*J*_{5',F} = 4.1 Hz, C-5'), 122.3 (d, ³*J*_{4',F} = 9.2 Hz, C-4'), 119.6 (d, ²*J*_{3',F} = 25.1 Hz, C-3'), 117.2 (d, *²J*_{1',F} = 12.0 Hz, C-1'), 62.1 (CH₂), 55.6 (C-1 "), 14.0 (C-2") ppm. EI-MS: m/z (%) = 329 (100), 327 (99) [M⁺]; 256 (93), 258 (92) [M⁺-73]; 202 (34), 200 (43) [M⁺-129]. C₁₂H₁₁BrFN₃O₂ (328.14): calc. C 43.92, H 3.38, N 12.81; found C 43.74, H 3.50, N 12.68

**4-(4-Bromo-2-fluorophenyl)-1-(ethoxycarbonyl)methyl-1*H*-[1,2,3]triazole (16b):** White solid (29.52, 18%); m.p. 115-117 °C (cyclohexane/Et₂O). ¹H NMR (200 MHz, CDCl₃, 25 °C): δ = 8.17 (t, ³*J*_{5',6'} = ⁴*J*_{6',F} = 8.2 Hz, 1 H, H-6'), 8.18 (d, ⁵*J*_{5,F} = 3.6 Hz, 1 H, H-5), 7.41-7.33 (m, 2 H, H-3', H-5'), 5.25 (s, 2 H, CH₂), 4.29 (q, ³*J*_{1",2"} = 7.2 Hz, 2 H, H-1"), 1.31 (t, ³*J*_{1",2"} = 7.2 Hz, 3 H, H-2") ppm; ¹³C NMR (50 MHz, CDCl₃, 25 °C): δ =166.1 (CO), 158.7 (d, ¹*J*_{2',F} = 251.0 Hz, C-2'), 140.8 (C-4), 128.7(d, ³*J*_{6',F}=4.1 Hz, C-6'), 128.0 (d, ⁴*J*_{5',F} = 3.6 Hz, C-5'), 124.2 (d, ⁴*J*_{5,F} = 13.0 Hz, C-5), 121.8 (d, ³*J*_{4',F} = 9.5 Hz, C-4'), 119.3 (d, ²*J*_{3',F} = 24.6 Hz, C-3'), 117.4 (d, ²*J*_{1',F} = 12.8 Hz, C-1'), 62.5 (CH₂), 50.9 (C-1"), 14.0 (C-2") ppm. EI-MS: m/z (%) = 329 (35), 327 (35) [M⁺]; 229 (51), 227 (61) [M⁺-100]; 214 (100), 212 (68) [M⁺-115]. C₁₂H₁₁BrFN₃O₂ (328.14): calc. C 43.92, H 3.38, N 12.81; found C 44.22, H 3.45, N 12.54

**4-(*p*-Chlorophenyl)-2-(ethoxycarbonyl)methyl-5-phenyl-2*H*-[1,2,3]triazole (17a):** White solid (104.1 mg, 60%); m.p. 94-96° C (cyclohexane/AcOEt). ¹H NMR (200 MHz, CDCl₃, 25 °C): δ = 7.54-7.47 (m, 4 H, H_{*o*'}, H*ₒ*), 7.38-7.35 (m, 3 H, H_{*m*'}, H_{*p*'}), 7.32 (dt, ³*J*_{o,m} = 8.8 Hz, ⁴*J*_{m,m} = ⁵*J*_{o,m} = 2.2 Hz, 2 H, H*ₘ*), 5.17 (2 H, s, CH₂), 4.19 (q, ³*J*_{1',2'} = 7.0 Hz, 2 H, H-1'), 1.21 (t, ³*J*_{1',2'} = 7.0 Hz, 3H, H-2') ppm; ¹³C NMR (50 MHz, CDCl₃, 25 °C): δ 166.6 (CO), 145.6 (C-5), 144.4 (C-4), 134.4 (C*ₚ*), 130.4 (C*_{ipso'}*), 129.5 (C*ₒ*), 129.1 (C*ᵢₚₛₒ*), 128.7 (C*ₘ*), 128.6 (C*_{p',}* C*_{m'}*), 128.3 (C*_{o'}*), 62.1 (CH₂), 55.6 (C-1'), 14.1 (C-2') ppm. EI-MS: m/z (%) = 343 (41), 341 (95) [M⁺]; 270 (45), 268 (100) [M⁺-73]; 104 (50) [M⁺-237]. C₁₈H₁₆ClN₃O₂ (341.79): caldc. C 63.25, H 4.72, N 12.29; found C 62.99, H 5.01, N 12.08

### Synthesis of N-adamanthyl-1,2,3-triazoles (compounds 18-20). General procedure

A mixture of 1,2,3-triazole **4-6** (0.3 mmol) and 1-bromoadamantane (64.5 mg, 0.3 mmol) was stirred at 200 °C for 2 h. The mixture oily crude with different regioisomers were separated by column chromatography eluting with cyclohexane/AcOEt (20: 1) to get the isomers **18a-20a** then eluting with cyclohexane/AcOEt (85:15) to obtain the isomer 18b.

**2-Adamantyl-4-(4-bromo-2-fluorophenyl)-2*H*-[1,2,3]triazole (18a): Yellow solid** (58.7 mg, 52%); m.p. 102-104 °C (cyclohexane/AcOEt). ¹H NMR (200 MHz, CDCl₃, 25 °C): δ = 7.94 (d, ⁵*J*_{5,F} = 4.0 Hz, 1 H, H-5), 7.93 (dd, ⁴*J*_{6",F} = 9.0 Hz, ³*J*_{6",5"} = 8.0 Hz, 1 H, H-6"), 7.38-7.31 (m, 2 H, H-5", H-3"), 2.33 (sw, 6 H, H-2'), 2.28 (sw, 3 H, H-3'), 1.83-180 (m, 6 H, H-4') ppm; ¹³C NMR (75 MHz, CDCl₃, 25 °C): δ = 159.9 (d, ¹*J*_{2",F} = 253.9 Hz, C-2"), 140.5 (C-4), 133.2 (d, ⁴*J*_{5,F} = 11.6 Hz, C-5), 129.7 (d, ³*J*_{6",F} = 4.5 Hz, C-6"), 128.2 (d, ⁴*J*_{5",F} = 3.5 Hz, C-5"), 121.9 (d, ³*J*_{4",F} *=* 9.6 Hz, C-4"), 119.9 (d, ²*J*_{3",F} = 25.1 Hz, C-3"), 118.6 (d, ²*J*_{1",F} = 13.0 Hz, C-1"), 63.5 (C-1'), 42.8 (C-2'), 36.4 (C-4'), 30.1 (C-3') ppm. EI-MS: m/z (%) = 377 (60), 375 (60) [M⁺]; 135 (100) [M⁺-240]. C₁₈H₁₉BrFN₃ (376.27): calc. C 57.46, H 5.09, N 11.17; found C 56.97, H 5.50, N 10.65

**1-Adamantyl-4-(4-bromo-2-fluorophenyl)-1*H* [1,2,3]triazole (18b):** Yellow solid (31.6 mg, 28%); m.p. 138-140 °C (cyclohexane/AcOEt). ¹H NMR (200 MHz, CDCl₃, 25 °C): δ = 8.13 (dd, ⁴*J*_{6",F} = 8.6 Hz, ³*J*_{6",5"} = 8.0 Hz, 1 H, H-6"), 7.89 (d, ⁵*J*_{5,F} = 3.6 Hz, 1 H, H-5), 7.34-7.19 (m, 2 H, H-3", H-5"), 2.23 (sw, 9 H, H-2', H-3'), 1.75 (sw, 6 H, H-4') ppm. ¹³C NMR (75 MHz, CDCl₃, 25 °C): δ = 159.1 (d, ¹*J*_{2",F} = 251.8 Hz, C-2"), 139.8 (d, ³*J*_{4,F} = 2.8 Hz, C-4), 129.2 (d, ³*J*_{6",F} = 4.5 Hz, C-6"), 128.4 (d, ⁴*J*_{5",F} = 3.5 Hz, C-5"), 121.6 (d, ⁴*J*_{5,F} = 9.8 Hz, C-5), 119. 8 (d, ³*J*_{4",F} = 3.5 Hz, C-4"), 119.5 (d, ²*J*_{1",F} = 9.0 Hz, C-1"), 118.7 (d, ²*J*_{3",F} = 13.1 Hz, C-3"), 60.3 (C-1'), 42.8 (C-2'), 36.4 (C-4'), 30.0 (C-3') ppm; EI-MS: m/z (%) = 377 (21), 375 (21) [M⁺]; 135 (100) [M⁺-240]. C₁₈H₁₉BrFN₃ (376.27): calc. C 57.46, H 5.09, N 11.17; found C 57.33, H 5.30, N 11.24

**2-Adamantyl-4-(*p*-chlorophenyl)-5-phenyl-2*H*-[1,2,3]triazole (19a):** White solid (101.7 mg, 87%); m.p. 145-147° C (cyclohexane/Et₂O). ¹H NMR (300 MHz, CDCl₃, 25 °C): δ = 7.43-7.36 (m, 4 H, H*ₒ*, H*ₒ*), 7.34-7.25 (m, 5 H, H*ₘ*, H*_{p'}*, H*ₘ*), 2.30 (sw, 6 H, H-2'), 2.19 (sw, 3 H, H-3'), 1.73 (sw, 6 H, H-4') ppm; ¹³C NMR (75 MHz, CDCl₃, 25 °C): δ = 143.4 (C-5), 142.1 (C-4), 133.8 (C*ₚ*), 131.4 (*C*_{*ipso*'}), 130.2 (C*ᵢₚₛₒ*), 129.5 (C*ₒ*), 128.6 (C*ₘ*), 128.5 (C*ₘ*), 128.3 (C*ₒ*), 128.1 (C_{*p*'}), 63.0 (C-1'), 42.4 (C-2'), 36.0 (C-4'), 29.6 (C-3') ppm. EI-MS: m/z (%) = 391 (49), 389 (100) [M⁺]; 135 (84) [M⁺-254]. C₂₄H₂₄ClN₃ (389.92): caldc. C 73.93, H 6.20, N 10.78; found C 73.78, H 6.42, N 10.79

**2-Adamantyl-4-(2,4-dichlorophenyl)-5-*p*-tolyl-2*H*-[1,2,3]triazole (20a):** White solid (108.8 mg, 83%); m.p. 142-144° C (cyclohexane). ¹H NMR (300 MHz, CDCl₃, 25 °C): δ = 7.45 (d, ⁴*J*_{3",5"} = 2.4 Hz, 1 H, H-3"), 7.37 (d, ³*J*_{5",6"} = 8.5 Hz, 1 H, H-6"), 7.34 (d, ³*J*_{o,m} = 8.0 Hz, 2 H, H*ₒ*), 7.31 (dd, ³*J*_{5",6"} = 8.5 Hz, ⁴*J*_{3",5"} = 2.4 Hz, 1 H, H-5"), 7.10 (d, ³*J*_{o,m} = 8.0 Hz, 2 H, H*ₘ*), 2.36-2.37 (m, 6 H, H-2'), 2.32 (s, 3 H, CH₃), 2.27 (sw, 3 H, H-3'), 1.80-1.78 (m, 6 H, H-4') ppm; ¹³C NMR (75 MHz, CDCl₃, 25 °C): δ = 144.2 (C-5), 139.7 (C-4),137.8 (C*ₚ*), 133.2 (C-1"), 132.7 (C-2"), 132.6 (C-4"), 131.9 (C-3"), 131.0 (C6"), 129.8 (C5"), 129.2 (C*ₘ*), 128.3 (C*ᵢₚₛₒ*), 126.6 (C*ₒ*), 63.2 (C-1'), 42.4 (C-2'), 36.0 (C-4'), 29.6 (C-3'), 21.3 (CH₃). EI-MS: m/z (%) = 441 (28), 439 (72), 437 (95) [M⁺]; 135 (100) [M⁺-302]. C₂₅H₂₅C1₂N₃ (438.45): caldc. C 68.49, H 5.75, N 9.59; found 68.83, H 5.91, N 9.58

### Synthesis of compounds N-carbamoyl-1,2,3-triazoles (compounds 21-26). General procedure

To a solution of amine (0.75 mmol) in dry CH₂Cl₂ (0.50 mL) was added dropwise under nitrogen a solution of trimethylaluminium in heptane 2M (0.38 mL, 0.75 mmol). The mixture was stirred at room temperature for 1 h. **16a-17a** (0.3 mmol) in dry CH₂Cl₂ (0.5 mL) was added under nitrogen and the solution was warmed to 45° C for 2 h. The reaction mixture was raised to 0 °C and poured carefully (exothermic reaction) into HCl (2N, 4.68 mL). The resultant mixture was stirred at 45 °C for 0.5 h. The aqueous layer was then extracted with 3 x 10 mL CH₂Cl₂. The combined organic extracts were dried over Na₂SO₄ and the oily residue was purified by column chromatography eluting with cyclohexane/AcOEt (1:1). RMN signals of **21-24** were described to majority conformer.

**4-(4-Chlorophenyl)-5-phenyl-2-(piperidin-1-yl-carbamoyl)methyl-2*H-*[1,2,3]triazole (21):** White solid (45.1 mg, 38%); m.p. 204-206 °C (toluene). ¹H NMR (500 MHz, DMSO-d₆, 25 °C): δ = 9.03 (s, 1 H, NH), 7.49-7.38 (m, 9 H, Hₐᵣ), 5.46 (s, 2 H, CH₂), 3.02 (sw, 2 H, H-2ec'), 2.71-2.69 (m, 2 H, H-2ax'), 2.40 (sw, 1 H, H-4ec'), 1.56-1.52 (m, 5 H, H-3', H-4ax') ppm; ¹³C NMR (100 MHz, DMSO-d₆, 25 °C): δ = 168.1 (CO), 144.6 (C-5), 143.3 (C-4), 133.8 (C*ₚ*), 131.1 (C*ᵢₚₛₒ*), 130.2 (C*ₒ*), 129.5 (C*_{p'}*, C*ₘ*, C*_{m'}*), 129.3 (C*ᵢₚₛₒ*), 128.6 (C*ₒ*), 57.3 (C-2'), 56.2 (CH₂), 26.0 (C-3'), 23.4 (C-4') ppm. EI-MS: m/z (%) = 395 (9) [M⁺]; 99 (100) [M⁺-296]; 84 (87) [M⁺-311]. C₂₁H₂₂ClN₅O (395.89): calc. C 63.71, H 5.60, N 17.69; found C 63.23, H 6.13, N 17.24

**4-(4-Bromo-2-fluorophenyl)-2-(piperidin-1-yl-carbamoyl)methyl-2*H*-[1,2,3]triazole (22):** White solid (50.5 mg, 44%); m.p. 235-237 °C (toluene). ¹H NMR (400 MHz, CDCl₃, 25 °C): δ = 8.01 (d, ⁵*J*_{5,F} = 2.1 Hz, 1 H, H-5), 7.87 (t, ³*J*_{6',5'} = ⁴*J*_{6',F} = 8.3 Hz, 1 H, H-6'), 7.32 (d, ³*J*_{3',F} = 8.3 Hz, ³*J*_{5',6'} = 8.3 Hz, 2 H, H-3', H-5'), 6.35 (sw, 1 H, NH), 5.45 (s, 2 H, CH₂), 3.12 (sw, 2 H, H-2ec"), 2.68 (sw, 1 H, H-4ec"), 2.34 (sw, 2 H, H-2ax"), 1.72-1.63 (m, 5 H, H-3", H-4ax") ppm; ¹³C NMR (100 MHz, CDCl₃, 25 °C): δ = 167.9 (CO), 159.5 (d, ¹*J*_{2',F} = 254.0 Hz, C-2'), 142.1 (C-4), 134.5 (d, ⁴*J*_{5,F} = 11.4 Hz, C-5), 129.4 (d, ³*J*_{6',F} = 4.6 Hz, C-6'), 127.9 (d, ⁴*J*_{5',F} = 3.1 Hz, C-5'), 122.1 (d, ³*J*_{4',F} = 9.9 Hz, C-4'), 119.6 (d, ²*J*_{3',F} = 25.2 Hz, C-3'), 117.5 (d, ²*J*_{1',F} = 13.0 Hz, C-1'), 58.1 (C-2"), 55.6 (CH₂), 25.6 (C-3"), 22.8 (C-4") ppm. EI-MS: m/z (%) = 383, 381 (9) [M⁺]; 99 (100) [M⁺-283]; 83 (92) [M⁺-299]. C₁₅H₁₇BrFN₅O (382.23): calc. C 47.13, H 4.48, N 18.32; found C 46.97, H 4.77, N 18.18

**4-(4-Chlorophenyl)-5-phenyl-2-(morpholin-4-yl-carbamoyl)methyl-2*H-*[1,2,3]triazole (23):** White solid (83.5 mg, 70%); m.p. 230-232 °C (toluene). ¹H NMR (500 MHz, DMSO-*d₆*, 25 °C): δ = 9.15 (s, 1 H, NH), 7.49-7.42 (m, 9 H, Hₐᵣ), 5.55 (s, 2 H, CH₂), 3.77-3.52 (m, 4 H, H-3'), 2.95-2.63 (m, 2 H, H-2') ppm; ¹³C NMR (100 MHz, DMSO-*d₆,* 25 °C): δ =167.6 (CO), 143.9 (C-5), 142.6 (C-4), 133.1 (C*ₚ*), 130.4 (C*_{ipso'}*), 129.5 (C*_{p'},* C*ₒ*), 128.9 (C*ₘ*), 128.8 (C*_{m'}*), 128.6 (C*ᵢₚₛₒ*), 127.9 (C*_{o'}*), 65.9 (C-3'), 55.7 (C-2'), 55.5 (CH₂) ppm. EI-MS: m/z (%) = 399 (14), 397 (39) [M⁺]; 270 (23), 268 (61) [M⁺-129]; 104 [M⁺-293] (56); 101 [M⁺-296] (100); 86 [M⁺-311] (67). C₂₀H₂₀ClN₅O₂ (397.86): calc. C 60.38, H 5.07, N 17.60; found C 60.15, H 5.31, N 17.43

**4-(4-Bromo-2-fluorophenyl)-2-(morpholin-4-yl-carbamoyl)methyl-2*H-*[1,2,3]triazole (24):** White solid (73.8 mg, 64%); m.p. 254-256 °C (toluene). ¹H NMR (500 MHz, DMSO-*d*₆, 25 °C): δ = 9.10 (s, 1 H, NH), 8.16 (d, ⁵*J*_{5,F} = 3.3 Hz, 1 H, H-5), 7.85 (dd, ³*J*_{6',5'} = 8.3 Hz, ⁴*J*_{6',F} = 7.8 Hz, 1 H, H-6'), 7.73 (dd, ³*J*_{3',F} = 10.3 Hz, ⁴*J*_{3',5'} = 2.0 Hz, 1 H, H-3'), 7.53 (dd, ³*J*_{6',5'} = 8.3 Hz, ⁴*J*_{5',3}' = 2.0 Hz, 1 H, H-5'), 5.53 (s, 2 H, CH₂), 3.61-3.59 (m, 4 H, H-3"), 2.77-2.75 (m, 4 H, H-2") ppm; ¹³C NMR (125 MHz, DMSO-*d*₆, 25 °C): δ = 167.5 (CO), 158.8 (d, ¹*J*_{2',F} = 253.3 Hz, C-2'), 140.7 (C-4), 133.7 (d, ⁴*J*_{5,F} = 9.9 Hz, C-5), 129.4 (d, ³*J*_{6',F} = 4.1 Hz, C-6'), 128.3 (d, ⁴*J*_{5',F} = 3.1 Hz, C-5'), 121.6 (d, ³*J*_{4',F} = 9.8 Hz, C-4'), 119.7 (d, ²*J*_{3',F} = 24.4 Hz, C-3'), 117.3 (d, ³*J*_{1',F} = 13.0 Hz, C-1'), 65.9 (C-3"), 55.7 (C-2"), 55.6 (CH₂) ppm. EI-MS: m/z (%) = 385, 383 (4) [M⁺]; 300, 298 (7) [M-85]; 256, 254 (11) [M-129]; 202, 200 (16) [M-185]; 200, 198 (16) [M-183]; 101 (100) [M-283]. C₁₄H₁₅BrFN₅O₂ (384.20): calc. C 43.77, H 3.94, N 18.23; found C 43.94, H 3.90, N 18.62

**4-(4-Chlorophenyl)-5-phenyl-2-(cyclohexyl-carbamoyl)methyl-2*H*-[1,2,3]triazole (25):** White solid (66.3 mg, 56%); m.p. 192-194 °C (cyclohexane). ¹H NMR (500 MHz, CDCl₃, 25 °C): δ =7.52-7.50 (m, 2 H, H_{*o*'}), 7.48 (d, ³*J*_{o,m} = 8.3 Hz, 2 H, H*ₒ*), 7.39-7.38 (m, 2 H, H_{*m*'}, H*ₚ*) 7.33 (d, ³*J*_{o,m} = 8.3 Hz, 2 H, H*ₘ*), 6.14 (d, ³*J*_{NH,1ax'} = 6.6 Hz, 1 H, NH), 5.14 (s, 2 H, CH₂), 3.82 (tq, ³*J*_{1ax',2ax'} = 10.2 Hz, ³*J*_{1ax',2eq'} = ³*J*_{NH,1ax'} = 6.6 Hz, 1 H, H-1ax'), 1.88 (m, 2 H, H-2eq'), 1.63 (m, 2 H, H-2ax'), 1.57 (m, 1 H, H-4eq'), 1.36 (m, 2 H, H-3eq'), 1.17 (m, 3 H, H-3ax', H-4ax') ppm; ¹³C NMR (50 MHz, CDCl₃, 25 °C): δ = 164.3 (CO), 145.8 (C-5), 144.5 (C-4), 134.7 (C*ₚ*), 130.0 (C*_{ipso'}*), 129.4 (C*ₒ*), 128.9 (C*ᵢₚₛₒ, Cₘ),* 128.7 (C*_{p'}*, C*_{m'}*), 128.2 (C*_{o'}*), 57.7 (CH₂), 48.4 (C1'), 32.6 (C-2'), 25.3 (C-4'), 24.5 (C-3') ppm. EI-MS: m/z (%) = 396 (36), 394 (82) [M⁺]; 314 (27), 312 (62) [M⁺-82]; 271 (42), 269 (96) [M⁺-182]; 214 (44), 212 (100) [M⁺-182]; 138 (48) [M⁺-257]; 104 (70) [M⁺-291]. C₂₂H₂₃ClN₄O (394.90): calc. C 66.91, H 5.87, N 14.19; found C 66.82, H 6.08, N 14.02

**4-(4-Bromo-2-fluorophenyl)-2-(cyclohexyl-carbamoyl)methyl-2*H*-[1,2,3]triazole (26):** White solid (48.0 mg, 42%); m.p. 228-230 °C (toluene). ¹H NMR (500 MHz, DMSO-*d₆*, 25 °C): δ = 8.25 (d, ³*J*_{NH,1"} = 7.5 Hz, 1 H, NH), 8.15 (d, ⁵*J*_{5,F} = 3.4 Hz, 1 H, H-5), 7.85 (dd, ⁴*J*_{6',F} 9.2 Hz, ³*J*_{6',5'} = 8.3 Hz, 1 H, H-6'), 7.72 (d, ³*J*_{3',F} = 10.3 Hz, 1 H, H-3'), 7.52 (d, ³*J*_{6',5'} = 8.3 Hz, 1H, H-5'), 5.15 (s, 2 H, CH₂), 3.53-3.52 (m, 1 H, H-1"), 1.76-1.51 (m, 4 H, H-2"), 1.28-1.06 (m, 6 H, H-3", H-4"); ¹³C NMR (125 MHz, DMSO-*d₆*, 25 °C): δ =164.0 (CO), 158.9 (d, ¹*J*_{2',F} = 253.3 Hz, C-2'), 140.8 (C-4), 133.8 (d, ⁴*J*_{5,F} = 9.9 Hz, C-5), 129.5 (d, ³*J*_{6',F} = 3.8 Hz, C-6'), 128.3 (d, ⁴*J*_{5',F} = 3.1 Hz, C-5'), 121.7 (d, ³*J*_{4',F} = 9.9 Hz, C-4'), 119.7 (d, ²*J*_{3',F} = 25.2 Hz, C-3'), 117.2 (d, ³*J*_{1',F} = 13.0 Hz, C-1'), 56.9 (CH₂), 47.8 (C-1"), 32.3 (C-2"), 25.1 (C-4"), 24.4 (C-3") ppm. EI-MS: m/z (%) = 382 (27), 380 (27) [M⁺]; 257 (98), 255 (98) [M⁺-126]; 200 (69), 198 (69) [M⁺-182]; 83 (68) [M⁺-283]; 55 (78) [M⁺-327]. C₁₆H₁₈BrFN₄O (381.24): calc. C 50.41, H 4.76, N 14.70; found C 50.72, H 4.58, N 14.95.

### BIOLOGICAL ACTIVITY

### In-vitro determination of affinity to CB1/CB2-Receptors

Compounds 10a, 10c, 11a, 13a, 14b, 15a, y 19a synthesized according to the procedures described above were tested for their affinity to CB1/CB2 receptors.

### Pharmacological Methods

The in-vitro determination of the affinity of the inventive substituted pyrazoline compounds to CB₁/CB₂-receptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human CB₁ and CB₂ receptors of Receptor Biology, Inc. are used. The radioligand used for both receptors is [³H]-CP55940. The respective parts of the description are hereby incorporated by reference and forms part of the present disclosure.

### Pharmacological Data

The affinity of the inventive substituted pyrazoline compounds to CB₁/CB₂ receptors was determined as described above. Some of the values obtained are given in the following table I:

**Table I:**

| Compound | CB₁-Receptor [³H]-CP55940 |
|---|---|
| | % Inhibition 10⁻⁶ M |
| **10a** | 22.8 |
| **10c** | 21.1 |
| **11a** | 29.8 |
| **13a** | 45.6 |
| **14b** | 24.6 |
| **15a** | 26.6 |
| **19a** | 23.1 |

## Claims

1. Use of a compound of formula I: 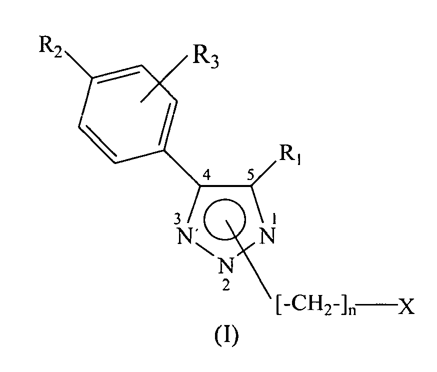 wherein:
R₁ is selected from hydrogen and substituted or unsubstituted aryl;
R₂ is selected from halogen, lower alkyl and lower alkoxy;
R₃ is selected from hydrogen and halogen;
X is selected from hydrogen, substituted or unsubstituted cycloalkyl, substituted or unsubstituted aryl, COOR' and CONHR"; wherein R' is a lower alkyl and R" is a cycloalkyl or heterocyclyl group;
n is selected from 0, 1, 2, 3, 4, 5, 6, 7, and 8,
or a pharmaceutically acceptable salt, isomer, prodrug or solvate thereof, in the manufacture of a medicament for the treatment or prophylaxis of cannabinoid receptor mediated disease or condition.

2. Use according to claim 1 for the treatment or profilaxis of the central nervous system, disorders of the immune system, disorders of the cardiovascular system, disorders of the endocrinous system, disorders of the respiratory system, disorders of the gastrointestinal tract or reproductive disorders.

3. Use according to claim 2 for the treatment or profilaxis of psychosis.

4. Use according to claim 1 for the treatment or profilaxis of food intake disorders, preferably bulimia, anorexia, cachexia, obesity, type II diabetus mellitus, more preferably obesity.

5. Use according to claim 1 for the treatment or profilaxis of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

6. Use according to claim 1 for the treatment or profilaxis of cancer, preferably selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the group consisting of colon cancer, bowel cancer and prostate cancer.

7. Use according to claim 1 for the treatment or profilaxis of a disorder selected from the group consisting of bone disorders, preferably osteoporosis, more preferably osteoporosis associated with a genetic predisposition, sex hormone deficiency or ageing, cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus and pain.

8. Use according to claim 1 for potentiating the analgesic effect of narcotic and non-narcotic analgesics and for influencing intestinal transit.

9. A compound of formula (I): 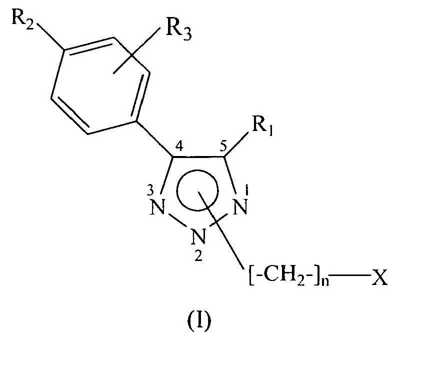 wherein:
R₁ is selected from hydrogen and substituted or unsubstituted aryl;
R₂ is selected from halogen, lower alkyl and lower alkoxy;
R₃ is selected from hydrogen and halogen;
X is selected from hydrogen, substituted or unsubstituted cycloalkyl, substituted or unsubstituted aryl, COOR' and CONHR"; wherein R' is a lower alkyl and R" is a cycloalkyl or heterocyclyl group;
n is selected from 0, 1, 2, 3, 4, 5, 6, 7, and 8,
or a pharmaceutically acceptable salt, isomer, prodrug or solvate thereof,
with the proviso that formula (I) is not:
4-(4-isopropoxy-phenyl)-2-ethyl-2*H*[1,2,3]triazole;
4-(4-hexyloxy-phenyl)-2-ethyl-2*H*-[1,2,3]triazole;
4-(4-chloro-phenyl)-2-ethyl-2*H*-[1,2,3]triazole;
4-(4-methoxy-phenyl)-2-methyl-2*H*[1,2,3]triazole;
4-(4-chloro-phenyl)-2-methyl-2*H*[1,2,3]triazole;
4-(4-bromo-phenyl)-2-methyl-2*H*-[1,2,3]triazole;
4-(4-fluoro-phenyl)-2-methyl-2*H*-[1,2,3]triazole
4-(3,4-dichloro-phenyl)-2-methyl-2*H*[1,2,3]triazole;
4-(2,4-difluoro-phenyl)-2-methyl-2*H*-[1,2,3]triazole;
4-(4-bromo-phenyl)-1-ethyl-1*H*-[1,2,3]triazole;
4-(4-isopropoxy-phenyl)-1-ethyl-1*H*-[1,2,3]triazole;
4-(4-butoxy-phenyl)-1-ethyl-1*H*-[1,2,3]triazole;
4-(4-hexyloxy-phenyl)-1-ethyl-1*H*-[1,2,3]triazole;
4-(4-bromo-phenyl)-1-methyl-1*H*-[1,2,3]triazole.

10. A compound according to claim 9 wherein R₁ is hydrogen, phenyl or 4-methyl-phenyl.

11. A compound according to claims 9 or 10 wherein R₂ is chloro, bromo, fluor, methyl or methoxyl.

12. A compound according to any of claims 9 to 11 wherein R₃ is hydrogen, chloro or fluor.

13. A compound according to any of claims 9 to 12 wherein X is hydrogen, adamanthyl, substituted or unsubstituted phenyl, COOR' or CONHR", wherein R' is preferably ethyl and R" is preferably cyclohexyl, pyridine or morpholine.

14. The compound according to any of claims 9 to 13 which is:
4-(*p*-tolyl)-1*H*(2*H*)-[1,2,3]triazole
4-(p-methoxyphenyl)-1*H*(2*H*)-[1,2,3]triazole
4-(4-bromo-2-fluorophenyl)-1*H*(2*H*)-[1,2,3]triazole
4-(*p*-chlorophenyl)-5-phenyl-1*H*(2*H*)-[1,2,3]triazole
4-(2,4-dichlorophenyl)-5-*p*-tolyl-1*H*(2*H*)-[1,2,3]triazole
2-Pentyl-4-*p*-tolyl-2*H*-[1,2,3]triazole
1-Pentyl-4-*p*-tolyl-1*H*-[1,2,3]triazole
1-Pentyl-5-*p*-tolyl-1*H*-[1,2,3]triazole
4-(*p*-Methoxyphenyl)-2-pentyl-2*H*-[1,2,3]triazole
4-(*p*-Methoxyphenyl)-1-pentyl-1*H*-[1,2,3]triazole
4-(4-Bromo-2-fluorophenyl)-2-pentyl-2H-[1,2,3]triazole
4-(4-Bromo-2-fluorophenyl)-1-pentyl-1*H*-[1,2,3]triazole
4-(*p*-Chlorophenyl)-2-pentyl-5-phenyl-2*H*-[1,2,3]triazole
4-(*p*-Chlorophenyl)-1-pentyl-5-phenyl-1*H*-[1,2,3]triazole
5-(*p*-Chlorophenyl)-1-pentyl-4-phenyl-1*H*-[1,2,3]triazole
4-(2,4-Dichlorophenyl)-2-pentyl-5-*p*-tolyl-2*H*-[1,2,3 ]triazole
4-(*p*-Chlorophenyl)-2-heptyl-5-phenyl-2*H*-[1,2,3]triazole
4-(*p*-Chlorophenyl)-1-heptyt-5-phenyl-1*H*-[1,2,3]triazole
5-(*p*-Chlorophenyl)-1-heptyl-4-phenyl-1*H*-[1,2,3]triazole
2-(*p*-Bromobenzyl)-4-(*p*-chlorophenyl)-5-phenyl-2*H*-[1,2,3]triazole
1-(*p*-Bromobenzyl)-4-(*p*-chlorophenyl)-5-phenyl-1*H*-[1,2,3]triazole
1-(*p*-Bromobenzyl)-5-(*p*-chlorophenyl)-4-phenyl-1*H*-[1,2,3]triazole
2-Benzyl-4-(*p*-chlorophenyl)-5-phenyl-2*H*-[1,2,3]triazole
1-Benzyl-4-(*p*-chlorophenyl)-5-phenyl-1*H*-[1,2,3]triazole
1-Benzyl-5-(*p*-chlorophenyl)-4-phenyl-1*H* [1,2,3]triazole
2-Benzyl-4-(4-bromo-2-fluorophenyl)-2H-[1,2,3]triazole
1-Benzyl-4-(4-bromo-2-fluorophenyl)-1*H*-[1,2,3]triazole
4-(4-Bromo-2-fluorophenyl)-2-(ethoxycarbonyl)methyl-2*H*[1,2,3]-triazole
4-(4-Bromo-2-fluorophenyl)-1-(ethoxycarbonyl)methyl-1*H*-[1,2,3]triazole
4-(*p*-Chlorophenyl)-2-(ethoxycarbonyl)methyl-5-phenyl-2*H*-[1,2,3] triazole
2-Adamantyl-4-(4-bromo-2-fluorophenyl)-2*H*-[1,2,3]triazole
1-Adamantyl-4-(4-bromo-2-ftuorophenyl)-1*H*-[1,2,3]triazote
2-Adamantyl-4-(*p*-chlorophenyl)-5-phenyl-2*H*-[1,2,3]triazole
2-Adamantyl-4-(2,4-dichlorophenyl)-5-*p*-tolyl-2*H*-[1,2,3]triazole
4-(4-Chlorophenyl)-5-phenyl-2-(piperidin-1-yl-carbamoyl)methyl-2*H*-[1,2,3]triazole
4-(4-Bromo-2-fluorophenyl)-2-(piperidin-1-yl-carbamoyl)methyl-2*H*-[1,2,3]triazole
4-(4-Chlorophenyl)-5-phenyl-2-(morpholin-4-yl-carbamoyl)methyl-2*H*-[1,2,3]triazole
4-(4-Bromo-2-fluorophenyl)-2-(morpholin-4-yl-carbamoyl)methyl-2*H*-[1,2,3]triazole
4-(4-Chlorophenyl)-5-phenyl-2-(cyclohexyl-carbamoyl)methyl-2*H*-[1,2,3]triazole
4-(4-Bromo-2-fluorophenyl)-2-(cyclohexyl-carbamoyl)methyl-2*H*-[1,2,3]triazole
or a pharmaceutically acceptable salt, prodrug or solvate thereof.

15. A process for the preparation of a compound of formula I, as defined in claims 9 to 14 or a salt, isomer, prodrug or solvate thereof, which comprises:
a) cycloaddition of tri-n-butyltin azide with a compound of formula (II): 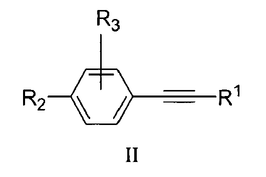 to give a compound of formula (III): 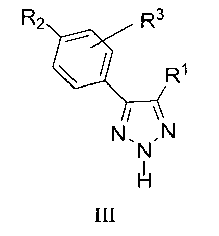
wherein:
R₁ is selected from hydrogen and substituted or unsubstituted phenyl;
R₂ is selected from halogen, lower alkyl and lower alkoxy;
R₃ is selected from hydrogen and halogen.

16. The process according to claim 15 which further comprises the alkylation of the compound of formula (III), with a compound of formula (IV): 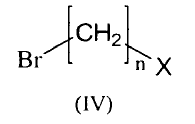 wherein
X is selected from hydrogen, substituted or unsubstituted cycloalky, substituted or unsubstituted aryl, COOR' and CONHR", wherein R' is a lower alkyl and R" is a cycloalkyl or heterocyclyl group; and
n is selected from 0, 1, 2, 3, 4, 5, 6, 7, and 8,
with the proviso that when n=0, X is not hydrogen,
to give a compound of formula (I).

17. The process according to claims 15 and 16 which further comprises the reaction between a compound of formula (I) wherein X is COOR' with an amine NH₂R" to give a compound of formula (I) wherein X is CONHR", being R' a lower alkyl and R" a cycloalkyl or heterocyclyl group.

18. A pharmaceutical composition which comprises a compound as defined in any of claims 9 to 14 or a pharmaceutically acceptable salt, isomer, prodrug or solvate thereof, and a pharmaceutically acceptable carrier, adjuvant or vehicle.
